(19) Europäisches Patentamt
European Patent Office
Office européen des brevets

(11) Numéro de publication : **0 296 059 B1**

# (12) FASCICULE DE BREVET EUROPEEN

(45) Date de publication du fascicule du brevet : **18.03.92 Bulletin 92/12**

(51) Int. Cl.$^5$ : **C07K 5/08,** A61K 37/02

(21) Numéro de dépôt : **88401476.2**

(22) Date de dépôt : **15.06.88**

(54) **Nouveaux tripeptides à structure polycyclique azotée, leur procédé de préparation et les compositions pharmaceutiques qui les contiennent.**

(30) Priorité : **16.06.87 FR 8708350**

(43) Date de publication de la demande : **21.12.88 Bulletin 88/51**

(45) Mention de la délivrance du brevet : **18.03.92 Bulletin 92/12**

(84) Etats contractants désignés : **AT BE CH DE ES FR GB GR IT LI LU NL SE**

(56) Documents cités :
**EP-A- 0 190 058**
**DE-A- 2 343 034**
**FR-A- 2 453 136**

(73) Titulaire : **ADIR ET COMPAGNIE**
**1 rue Carle Hébert**
**F-92415 Courbevoie Cédex (FR)**

(72) Inventeur : **Vincent, Michel**
**8 allée du Prunier Hardy**
**F-92220 Bagneux (FR)**
Inventeur : **Remond, Georges**
**9 avenue des Etats-Unis**
**F-78000 Versailles (FR)**
Inventeur : **Portevin, Bernard**
**6 rue Frédéric Passy**
**F-78320 Elancourt (FR)**
Inventeur : **Cudennec, Claude**
**23 bis avenue de Circourt**
**F-78170 La Celle St-Cloud (FR)**

EP 0 296 059 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux tripeptides, leur préparation et les compositions pharmaceutiques qui les contiennent.

On connaît de nombreux peptides naturels ou synthétiques, modificateurs de la réponse biologique et, notamment la tuftsine, tétrapeptide naturel de formule Thr - Lys - Pro - Arg.

Un analogue de la tuftsine dans lequel la thréonine est remplacée par un radical oxazolidinone carboxy a été décrit par Y. STABINSKY et al (Int. J. Peptide, Protein Research, 1978 ; 12 ; 130-138). Toutefois, ce dérivé ne retient environ que la moitié de l'activité de la tuftsine.

La demande de brevet européen n° 0 190 058 décrit des analogues de la tuftsine dans lesquels la proline est remplacée par une structure polycyclique azotée.

Toutefois, tous ces composés, selon des travaux récents (V.A NAJJAR ; Annals of the New York Academy of Science ; Volume 419 - Antineoplastic, immunogenic and other effects of the tetrapeptide tuftsine, New York Academy of Sciences, New York - 1983) possèdent l'inconvénient de subir l'action d'une aminopeptidase spécifique qui scinde la tuftsine ou ses homologues :

– d'une part en un radical thréonyle,

– d'autre part en un tripeptide (Lys - Pro - Arg, dans le cas de la tuftsine) qui est un inhibiteur de l'activité du tétrapeptide de départ.

Cette aminopeptidase rend donc assez rapidement inactifs la tuftsine et ses dérivés.

Toutefois, cette aminopeptidase ne peut agir que sur des composés présentant simultanément un groupement aminé en $\alpha$ du groupement carbonyle de la liaison peptidique impliquant le groupement aminé de la lysine, c'est-à-dire la structure suivante :

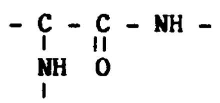

(a)

La demanderesse a maintenant découvert de nouveaux composés dont le niveau d'activité est supérieur à celui de la tuftsine mais qui ne possèdent plus la structure tétrapeptidique, donc dépourvus de la structure (a) ; en effet la thréonine a été remplacée par une structure qui n'est pas un $\alpha$amino acide, laquelle est greffée sur le reste lysyle. Ces nouveaux tripeptides offrent donc la caractéristique particulièrement avantageuse de posséder un haut niveau d'activité tout en étant insensibles à l'action de l'aminopeptidase et, partant, de posséder une activité moins fugace et donc plus compatible avec une utilisation thérapeutique ; ceci se traduit, en outre, par des possibilités d'utilisation en thérapeutique à une posologie plus faible, avec des administrations successives plus espacées.

Plus spécifiquement, l'invention concerne des dérivés de formule générale :

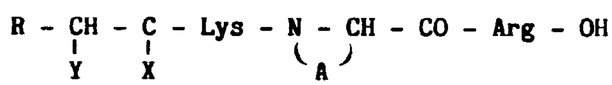

(I)

dans laquelle :

X représente

soit un atome d'oxygène,

soit deux atomes d'hydrogène.

Y représente

soit un atome d'hydrogène,

soit un groupement hydroxyle.

ou

Y représente un groupement amino à la condition que X représente deux atomes d'hydrogène.

R représente un atome d'hydrogène, un groupement alcoyle de 1 à 6 atomes de carbone en chaîne droite ou ramifiée éventuellement substitué par un ou plusieurs groupements hydroxy, amino, mercapto, méthylthio, carboxy ou aryle tel que phényle, pyridyle ou thiényle.

Lys et Arg représentent respectivement les restes lysyle et arginyle engagés dans des liaisons peptidiques,

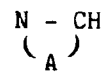

représente

* une structure bicyclique de formule

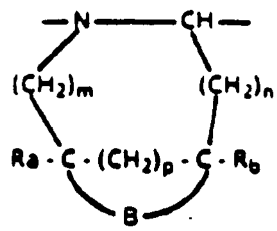

où

– m est égal à 1 ou zéro,

– n et p représentent zéro, 1 ou 2,

– Ra et Rb représentent un atome d'hydrogène ou peuvent former ensemble une liaison directe quand p = 0,

– B représente :

* une chaîne alkylène $(CH_2)q$ où q est égal à 2, 3 ou 4, ou

* une structure insaturée $( - CH = CH - )_2$ quand p = 0 et Ra et Rb forment ensemble une liaison, avec la réserve que la somme m, n, p et q est un nombre entier compris entre 3 et 6, ou

* la tétrahydro - 1, 2, 3, 4 - bétacarboline.

leurs énantiomères, épimères et diastéréoisomères,

ainsi que leurs sels d'addition à un acide ou une base pharmaceutiquement acceptable.

Actuellement, parmi les composés de formule (I), on préfère ceux dans lesquels la structure cyclique

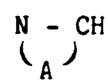

représente

l'indoline, l'isoindoline, la tétrahydroquinoléine, la tétrahydroisoquinoléine, la perhydroindole, le perhydroisoindole, la perhydroquinoléine, la perhydroisoquinoléine, le perhydrocyclopenta [b] pyrrole, l'aza - 2 bicyclo [2.2.2] octane, l'aza - 2 bicyclo [2.2.1] heptane, la tétrahydro - 1, 2, 3, 4 bétacarboline.

Parmi les acides que l'on peut ajouter aux composés de formule (I) pour former un sel d'addition, on peut citer, à titre d'exemple, les acides chlorhydrique, sulfurique, phosphorique, tartrique, malique, maléique, fumarique, oxalique, méthane-sulfonique, éthane-sulfonique, camphorique, citrique, etc...

Comme bases pouvant salifier les composés de formule (I) on pourra utiliser des hydroxydes de sodium, potassium, calcium ou d'aluminium, des carbonates de métaux alcalins ou alcalinoterreux ou des bases organiques comme la triéthylamine, la benzylamine, la diéthanolamine, la tert.butylamine, la dicyclohexylamine, l'arginine, etc...

L'invention s'étend aussi au procédé d'obtention des composés de formule (I),

caractérisé en ce que l'on condense un dérivé de formule (II) :

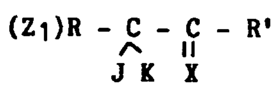

(II)

dans laquelle :

* R et X ont la même signification que dans la formule (I),

* R′ représente :

– soit un atome d'hydrogène, soit un groupement hydroxyle, lorsque X représente un atome d'oxygène,

– soit un atome d'halogène, préférentiellement un atome de brome, à la condition que dans ce cas X

représente deux atomes d'hydrogène,

* J représente un atome d'hydrogène ou un groupement hydroxyle à la condition que K représente un atome d'hydrogène, ou bien

J représente un groupement benzyloxycarbonylamino à la double condition que K et R′ représentent chacun un atome d'hydrogène, ou bien

J et K représentent ensemble un atome d'oxygène à la condition que X représente deux atomes d'hydrogène,

* ($Z_1$) représente un groupement protecteur des éventuels substituants aminés du radical R et en particulier le groupe benzyloxycarbonyle,

avec un dérivé de formule (III) :

**Lys (Z) - OtBu (III)**

dans laquelle :

(Z) représente un groupement protecteur du substituant ωaminé de la lysine, et en particulier le groupe benzyloxy carbonyle et tBu représente un groupement protecteur du groupement carboxyle, et en particulier le radical tertiobutyle,

en présence :

– d'un agent réducteur comme par exemple un hydrure mixte de métal alcalin, comme par exemple un borohydrure de métal alcalin ou bien un cyanoborohydure de métal alcalin lorsque R′ représente un atome d'hydrogène,

– d'un agent de couplage peptidique usuel, tel que la dicyclohexylcarbodiimide en présence d'hydroxybenzotriazole, lorsque R′ représente un groupement hydroxyle,

– d'un agent basique comme par exemple un sel de métal alcalin, comme par exemple un carbonate de métal alcalin tel que le carbonate de sodium lorsque R′ représente un atome d'halogène,

pour obtenir un dérivé de formule (IV) :

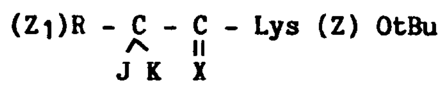

(IV)

dans laquelle R et X ont la même signification que dans la formule (I), J, K et ($Z_1$) la même signification que dans la formule (II) et (Z) et tBu la même signification que dans la formule (III),

que l'on déprotège en milieu acide au niveau de la fonction acide carboxylique de la lysine en un dérivé de formule (V) :

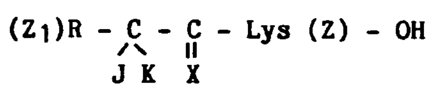

(V)

dans laquelle R et X ont la même signification que dans la formule (I), J, K et ($Z_1$) la même signification que dans la formule (II) et (Z) la même signification que dans la formule (III),

qui est ensuite condensé avec un dérivé de formule (VI) :

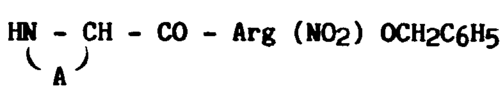

(VI)

dans laquelle A représente avec les atomes de carbone et d'azote auxquels il est attaché la même signification que dans la formule (I)

lui-même obtenu par condensation d'un dérivé de formule (VII) :

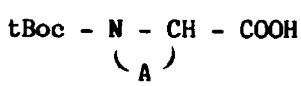

(VII)

dans laquelle A représente avec les atomes de carbone et d'azote auxquels il est attaché la même signification

que dans la formule (I) et tBoc représente le radical tertiobutoxycarbonyle,
obtenu comme décrit dans la demande de brevet européen n° 0 190 058,
avec le Nω - nitroarginate de benzyle (H - Arg ($NO_2$) - $OCH_2C_6H_5$) pour obtenir un dérivé de formule (VIII) :

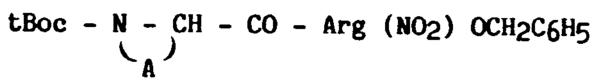

(VIII)

dans laquelle A, avec les atomes de carbone et d'azote auxquels il est attaché, a la même signification que dans la formule (I),
que l'on déprotège ensuite par l'acide trifluoroacétique selon la méthode décrite par B. GUTTE et K.B. MERRIFIELD (J. Am. Chem. Soc. 1969, 91, 501), en dérivé de formule (VI),
la condensation du dérivé de formule (V) avec le dérivé de formule (VI) permettant l'obtention d'un dérivé de formule (IX) :

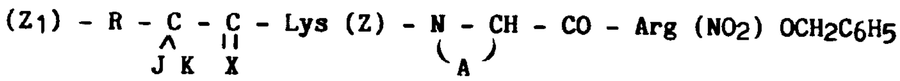

(IX)

dans laquelle A avec les atomes de carbone et d'azote auxquels il est attaché, R et X ont la même signification que dans la formule (I), J, K et ($Z_1$) la même signification que dans la formule (II) et (Z) la même signification que dans la formule (III),
qui est, lorsque J et K représentent ensemble un atome d'oxygène, soumis à l'action d'un hydrure mixte de métal alcalin tel que le borohydrure de sodium, le composé ainsi obtenu pouvant être, si on le désire, séparé en ses isomères par une technique classique de séparation comme la chromatographie sur colonne de silice, pour obtenir un composé de formule générale (IXa), cas particulier des dérivés de formule (IX), dans laquelle A avec les atomes de carbone et d'azote auxquels il est attaché, R, X, ($Z_1$), (Z) ont la même signification que dans la formule (IX), J représente un groupement hydroxyle et K représente un atome d'hydrogène,
dérivé de formule (IX) ou (IXa) que l'on déprotège par hydrogénolyse dans un solvant polaire acide en présence d'un catalyseur d'hydrogénation pour conduire à un dérivé de formule (I),
que l'on peut si on le désire :

- soit salifier par un acide ou une base pharmaceutiquement acceptable,
- soit séparer en ses isomères, puis, si nécessaire, salifier par un acide ou une base pharmaceutiquement acceptable.

Les dérivés de formule (IX) et (IXa) sont nouveaux et font partie de l'invention au même titre que les dérivés de formule (I), dont ils constituent les intermédiaires de synthèse.

Les composés de formule (I) sont doués de propriétés pharmacologiques intéressantes.

En particulier, on retrouve, chez ces dérivés, à un niveau supérieur ou, au moins comparable, les principales propriétés des composés de la demande de brevet européen n° 0 190 058.

Notamment, ces dérivés augmentent l'activité des cellules N.K. "Tueuses spontanées". Administrés à la souris porteuse d'un mélanome, ils inhibent de façon importante la croissance de ce mélanome. Ils assurent une promotion des défenses immunitaires chez des animaux infectés par des souches bactériennes pathogènes.

Ces activités sont liées aux propriétés immunomodulatrices des composés de l'invention qui trouvent leur application, en thérapeutique animale ou humaine, dans le traitement des cancers, des affections d'origine virale, bactérienne ou fongique, des maladies auto-immunes comme le lupus érythémateux ou l'arthrite rhumatoïde et plus généralement dans les maladies résultant d'une diminution ou d'une perturbation des réponses immunitaires naturelles de l'organisme animal ou humain.

En outre, la structure originale que possèdent les dérivés de la présente invention les rend insensibles à l'action d'une aminopeptidase spécifique, comme indiqué plus haut. Ceci rend leur activité moins fugace que celle des dérivés de l'art antérieur et donc nettement plus compatible avec une utilisation thérapeutique, permettant en particulier une posologie plus faible ainsi que des administrations successives plus espacées dans le temps.

L'invention a également pour objet les compositions pharmaceutiques contenant au moins un composé de formule générale (I), ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, on pourra citer plus particulièrement celles qui

conviennent pour l'administration orale, parentérale, per ou transcutanée, nasale, rectale, perlinguale, oculaire ou respiratoire, et notamment les préparations injectables, les aérosols, les gouttes oculaires ou nasales, les comprimés, les comprimés sublinguaux, les glossettes, les gélules, les capsules, les tablettes, les suppositoires, les crèmes, pommades, gels dermiques, etc...

La posologie utile varie selon l'âge et le poids du patient, la voie d'administration, la nature de l'indication thérapeutique et des traitements éventuellement associés et s'échelonne entre 1 microgramme et 1 gramme par prise ou par application.

Les exemples suivants illustrent l'invention, et ne la limitent en aucune façon.

Les produits de départ sont connus de la littérature.

Les points de fusion indiqués sont mesurés selon la technique micro-Köfler.

**EXEMPLE 1 :** (2S, 3R) AHPA - (S) Lys - (S) PHI - (S) Arg OH

AHPA - OH : Acide hydroxy - 2 amino - 3 phényl - 4 butanoïque (S)PHI - OH : Acide perhydroindole carboxylique - 2 (2S, 3aS, 7aS)

**STADE A :** tBoc (S) PHI - (S) Arg $(NO_2)OCH_2$ $C_6H_5$

En utilisant la méthode de W. KONIG et R. GEIGER, (Ber., 1970, 103, 788) coupler 0,004 mole de tBoc (S) PHI - OH, décrit dans la demande de brevet européen n° 0 190 058, avec 0,004 mole de (S) Nω nitroarginate de benzyle en utilisant le diméthylformamide anhydre comme solvant.

On obtient le tBoc (S) PHI - (S) Arg $(NO_2)$ $OCH_2C_6H_5$ utilisé tel quel dans le stade suivant.

**STADE B :** (S) PHI - (S) Arg $(NO_2)$ $OCH_2$ $C_6H_5$

En utilisant la méthode de déprotection par l'acide trifluoroacétique dans le chlorure de méthylène anhydre décrite par B. GUTTE et R.B MERRIFIELD (J. Am. Chem. Soc., 1969, 91, 501 ), on obtient quantitativement, à partir du tBoc (S) PHI - (S) Arg $(NO_2)$ $OCH_2C_6H_5$ préparé au stade précédent, le (S) PHI - (S) Arg $(NO_2)$ $OCH_2C_6H_5$ sous forme de trifluoroacétate.

**STADE C :** (Z) - (2S, 3R) AHPA - (S) Lys (Z) OtBu

Condenser le (Z) (2S, 3R) AHPA, décrit par R. NISHIZAWA et al (J. Med. Chem, 1977, 20, 4, 510-515) avec le dérivé (S) Lys (Z) - OtBu selon la méthode de W. KONIG et GEIGER (Chem. Ber., 1970, 103, 788) pour obtenir le (Z) (2S, 3R) AHPA - (S) Lys (Z) OtBu qui est utilisé tel quel au stade suivant.

**STADE D :** (Z) - (2S, 3R) AHPA - (S) Lys (Z) - OH

Le groupement carboxyle terminal du (Z) - (2S, 3R) - AHPA - (S) Lys (Z) OtBu est déprotégé par l'acide trifluoroacétique. Après mise à sec et concrétisation dans l'éther éthylique on obtient le (Z) - (2S, 3R) AHPA - (S) Lys (Z) - OH.

**STADE E :** (Z) - (2S, 3R) AHPA - (S) Lys (Z) - (S) PHI - (S) Arg $(NO_2)$ $OCH_2$ $C_6H_5$

Le (Z) - (2S, 3R) AHPA - (S) Lys (Z) - OH obtenu au stade précédent est couplé selon la méthode utilisée au stade A avec le (S) PHI - (S) Arg $(NO_2)$ $OCH_2$ $C_6H_5$ lui-même obtenu au stade B.

Le (Z) - (2S, 3R) AHPA - (S) Lys (Z) - (S) PHI - (S) Arg $(NO_2)$ $OCH_2$ $C_6H_5$ ainsi obtenu est purifié par chromatographie sur gel de silice (éluant chlorure de méthylène 95, méthanol : 5, Rf : 0,15).

**STADE F :** (2S, 3R) AHPA - (S) Lys - (S) PHI - (S) Arg OH

Dissoudre 650 mg de (Z) (2S, 3R) AHPA - (S) Lys (Z) - (S) PHI - (S) Arg $(NO_2)$ $OCH_2$ $C_6H_5$ obtenu au stade précédent dans 100 $cm^3$ d'acide acétique, ajouter 200 mg de charbon palladié à 10 % et hydrogéner pendant 20 heures sous une pression de 3 $Kg/cm^2$. Après filtration du catalyseur et évaporation de l'acide acétique, dissoudre le résidu dans 50 $cm^3$ d'eau, filtrer puis lyophiliser. Le (2S, 3R) AHPA - (S) Lys - (S) PHI - (S) Arg OH (360 mg) est obtenu sous forme de diacétate.

Spectrométrie de masse : (Spectre DCI $(NH_3)$)

M/Z :

571 : $[M + H - H_2O - HN = . = NH]^+$

124 : 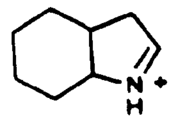

122 : $C_6H_5 - CH_2 - CH_2 - NH_3^+$

115 : $[Arg + H - H_2O - HN = . = NH]^+$

**EXEMPLE 2 :** (2R, 3R) AHPA - (S) Lys - (S) PHI - (S) Arg OH

En opérant comme dans l'exemple 1 à partir du (Z) (2R, 3R) AHPA (J. Med. Chem. 1977, 20, 510-515), on obtient successivement :

(Z) (2R, 3R) AHPA - (S) Lys (Z) - OtBu

(Z) (2R, 3R) AHPA - (S) Lys (Z) - OH

(Z) (2R, 3R) AHPA - (S) Lys (Z) - (S) PHI - (S) Arg ($NO_2$) $OCH_2C_6H_5$

(2R, 3R) AHPA - (S) Lys - (S) PHI - (S)Arg OH sous forme de diacétate.

Spectrométrie de masse : (spectre DCI ($NH_3$))

M/Z :

571 : $[M + H - H_2O - HN = . = NH]^+$

124 : 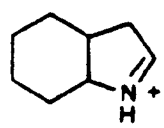

115 : $[Arg + H - H_2O - HN = . = NH]^+$

**EXEMPLE 3 :** (2S, 3S) AHPA - (S) Lys - (S) PHI - (S) Arg OH

En opérant comme dans l'exemple 1 à partir du (Z) - (2S, 3S) AHPA (J. Med. Chem. 1977, 20, 510-515), on obtient successivement :

(Z) (2S, 3S) AHPA - (S) Lys (Z) - OtBu

(Z) (2S, 3S) AHPA - (S) Lys (Z) - OH

(Z) (2S, 3S) AHPA - (S) Lys (Z) - (S) PHI - (S) Arg ($NO_2$) $OCH_2C_6H_5$

(2S, 3S) AHPA - (S) Lys - (S) PHI - (S) Arg OH sous forme de diacétate.

Spectrométrie de masse : (spectre DCI ($NH_3$))

M/Z :

613 : $[M + H - H_2O]^+$

589 : $[M + H - HN - . - NH]^+$

571 : $[M + H - H_2O - HN = . = NH]^+$

124 : 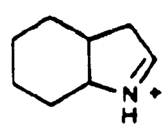

**EXEMPLE 4 :** (2R, 3S) AHPA - (S) Lys - (S) PHI - (S) Arg OH

En opérant comme dans l'exemple 1 à partir du (Z) - (2R, 3S) AHPA (J. Med. Chem. 1977, 20, 4, 510-515), on obtient successivement :

(Z) (2R, 3S) AHPA - (S) Lys (Z) - tBu

(Z) (2R, 3S) AHPA - (S) Lys (Z) - OH
(Z) (2R, 3S) AHPA - (S) Lys (Z) - (S) PHI - (S) Arg (NO2) - $OCH_2C_6H_5$
(2R, 3S) AHPA - (S) Lys - (S) PHI - (S) Arg OH sous forme de diacétate.
Spectrométrie de masse : (spectre DCI $(NH_3)$)
M/Z :
571 : $[M + H - H_2O - HN = . = NH]^+$

124 : 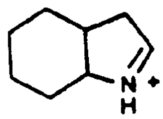

115 : $[Arg + H - H_2O - HN = . = NH]^+$

**EXEMPLE 5 :** (2S, 3R) AHPA - (S) Lys - (S) ABO - (S) Arg OH

ABO - OH : acide aza - 2 bicyclo [2.2.2] octane carboxylique - 3.

En opérant comme dans l'exemple 1 et en remplaçant au stade A le tBoc (S) PHI - OH par le tBoc (S) ABO - OH décrit dans la demande de brevet européen n° 0 190 058 on obtient successivement :

**STADE A :** tBoc (S) ABO - (S) Arg $(NO_2)$ - $OCH_2C_6H_5$
**STADE B :** (S) ABO - (S) Arg $(NO_2)$ - $OCH_2C_6H_5$

que l'on condense avec le (Z) - (2S, 3R) AHPA - (S) Lys (Z) - OH obtenu dans l'exemple 1 pour obtenir successivement :
(Z) (2S, 3R) AHPA - (S) Lys (Z) - (S) ABO - (S) Arg $(NO_2)$ - $OCH_2$ $C_6H_5$
(2S, 3R) AHPA - (S) Lys - (S) ABO - (S) Arg OH sous forme de diacétate.
Spectrométrie de masse : (spectre DCI : $(NH_3)$)
M/Z :
575 : $[M + H - HN = . = NH]^+$
557 : $[M + H - H_2O - HN = . = NH]^+$
120 : $[C_6H_5 - CH_2 - CH = NH_2]^+$

**EXEMPLES 6 ET 7 :**

N [Hydroxy - 2(S) amino - 3(R) butyryl] - (S) Lys - (S) ABO - (S) Arg - OH (isomère α)
et
N [Hydroxy - 2(R) amino - 3(R) butyryl] - (S)Lys - (S) ABO - (S) Arg - OH (isomère β)

**STADE A :**

En remplaçant dans l'exemple 1 stades A et B le (Z) - (2S, 3R) AHPA par l'acide hydroxy - 2(R, S) (Z) - amino - 3(R) butyrique décrit par R. NISHIZAWA, T. SAINO (J. Med. Chem. 1977, 20, 4, 510-515) on obtient successivement :
N [Hydroxy - 2(R,S) (Z) - amino - 3(R) butyryl] - (S) Lys (Z) - OtBu
puis
N [Hydroxy - 2(R,S) (Z) - amino - 3(R) butyryl] - (S) Lys (Z) - OH

**STADE B :**

N [Hydroxy - 2(S) (Z) - amino - 3(R) butyryl] - (S) Lys (Z) - (S) ABO - (S) Arg $(NO_2)$ - $OCH_2C_6H_5$
et
N [Hydroxy - 2(R) (Z) amino - 3(R) butyryl] - (S) Lys (Z) - (S) ABO - (S) Arg $(NO_2)$ - $OCH_2C_6H_5$
Le N [Hydroxy - 2(R, S) (Z) - amino - 3(R) butyryl] - (S) Lys (Z) - OH obtenu au stade précédent est couplé selon la technique utilisée dans l'exemple 1 stade E avec le (S) ABO -(S) Arg $(NO_2)$ - $OCH_2C_6H_5$ (voir exemple 5) pour donner le N [Hydroxy - 2(R,S) (Z) - amino - 3(R) butyryl] - (S) Lys (Z) - (S) ABO - (S) Arg (NO2) - $OCH_2C_6H_5$. Les deux isomères sont séparés par chromatographie sur gel de silice en utilisant comme éluant un mélange chlorure de méthylène / méthanol (96/4).
Chromatographie couche mince :
support : silice Si 60 F 254.

solvant : acétate d'éthyle.
Isomère α Rf.: 0,19
Isomère β Rf.: 0,12

**STADE C :**

N [Hydroxy - 2(S) amino - 3(R) butyryl] - (S) Lys - (S) ABO - (S) Arg - OH
et
N [Hydroxy - 2(R) amino - 3(R) butyryl] - (S) Lys - (S) ABO - (S) Arg - OH

Chacun des isomères obtenu au stade précédent est traité selon la technique utilisée dans l'exemple 1 stade F pour obtenir :

**EXEMPLE 6 :**

N [Hydroxy - 2(S) amino - 3(R) butyryl] - (S) Lys - (S) ABO - (S) Arg - OH lyophilisé sous forme de diacétate.

**EXEMPLE 7 :**

N [Hydroxy - 2(R) amino - 3(R) butyryl] - (S) Lys - (S) ABO - (S) Arg - OH lyophilisé sous forme de diacétate.
Pour les deux isomères :
Chromatographie couche mince :
support : silice Si 60 F254
solvant:

| | |
|---|---|
| acétate d'éthyle: | 20 |
| pyridine: | 20 |
| eau: | 15 |
| acide acétique: | 5 |

Rf. : 0,17
Spectrométrie de masse : $[FAB]^+$
M/Z :
563 : $[ M + Na]^+$
541 : $[ M + H]^+$

110 : 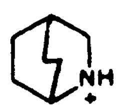

(Les spectres de masse $FAB^+$ des 2 isomères sont identiques)

**EXEMPLE 8 :** (3S, 4S) Sta - (S) Lys - (S) ABO - (S) Arg OH

Sta = statine = acide hydroxy - 3 amino - 4 méthyl - 6 heptanoïque

**STADE A :** (Z) - (3S, 4S) Sta

La (3S, 4S) statine préparée selon la méthode de D.H. RICH, E.T. SUN, A.S BOPARAI (J.O.C, 1978, 43, 18, 3624-3626) est transformée en (Z) - (3S, 4S) statine en utilisant la technique de M. BERGMANN et L. ZERVAS ( Ber. 1932, 65, 1192)

**STADE B :**

En remplaçant dans les exemples 6 et 7 l'acide hydroxy - 2(R, S) (Z) - amino - (3R) butyrique par le (Z) - (3S, 4S) Sta, on obtient successivement :
– (Z) - (3S, 4S) Sta - (S) Lys (Z) OtBu
– (Z) - (3S, 4S) Sta - (S) Lys (Z) OH
– (Z) - (3S, 4S) Sta - (S) Lys (Z) - (S) ABO - (S) Arg $(NO_2)$ $OCH_2C_6H_5$

– (3S, 4S) Sta - (S) Lys - (S) ABO - (S) Arg OH sous forme de diacétate
Spectrométrie de masse : (spectre DCI ($NH_3$))
M/Z :

537 : [M + H - H20 - HN = . = NH]$^+$

110 : 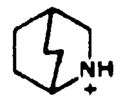

**EXEMPLE 9 :** (R,S) GABOB - (S) Lys - (S) ABO - (S) Arg OH

GABOB : Acide hydroxy -3 amino - 4 butyrique

En remplaçant dans l'exemple 8 la (3S, 4S) statine par le GABOB on obtient successivement :

(Z) - (R, S) GABOB
(Z) - (R, S) GABOB - (S) Lys (Z) OtBu
(Z) - (R, S) GABOB - (S) Lys (Z) OH
(Z) - (R, S) GABOB - (S) Lys (Z) - (S) ABO - (S) Arg ($NO_2$) - $OCH_2C_6H_5$
(R, S) GABOB - (S) Lys - (S) ABO - (S) Arg OH

lyophilisé sous forme de diacétate

Spectrométrie de masse : Spectre FAB$^+$
M/Z :

563 : [M + Na]$^+$
541 : [M + H]$^+$

115 : 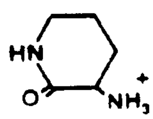

110 : 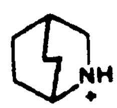

**EXEMPLE 10 :** (Phényl - 3 amino - 2(S) propyl) - (S) Lys - (S) ABO - (S) Arg - OH

STADE A : (Phényl - 3 (Z) - amino - 2(S) propyl) - (S) Lys (Z) - OtBu

Le (Z)(S) Phénylalaninal obtenu selon la méthode de R. NISHIZAWA, T. SAINO (J. Med. Chem. 1977, 20, 510-515) est soumis à amination réductrice en présence de cyanoborohydrure de sodium avec le (S) Lys (Z) - OtBu selon la technique de J. Martinez (J. Med. Chem. 1985, 28, 1874-1879). Le (phényl - 3 (Z) - amino - 2(S) propyl) - (S)Lys(Z) - OtBu, purifié par chromatographie sur gel de silice (éluant chlorure de méthylène, éthanol 95/5) est obtenu avec un rendement de 74 %.

Caractéristiques spectrales :

Infra-rouge :

νs NH : 3320 $cm^{-1}$
νs CO : 1720 $cm^{-1}$

**STADE B :** (Phényl - 3 (Z) - amino - 2(S) propyl) - (S) Lys (Z) - OH

15 g de (Phényl - 3 (Z) - amino - 2(S) propyl) - (S)Lys(Z) - OtBu obtenu au stade précédent sont soumis pendant 18 heures à température ambiante à l'action d'une solution 2N d'acide chlorhydrique dans l'acétate d'éthyle. Après mise à sec, reprise à l'éther, filtration et lavage à l'acétate d'éthyle on recupère avec un rendement de 66 % le (Phényl - 3 (Z) amino - 2(S) propyl) - (S) Lys (Z) - OH, sous forme de chlorhydrate.

Caractéristiques spectrales :

Infra-rouge : ν(C = O) : 1730 et 1680 $cm^{-1}$

**STADE C :** (Phényl - 3 (Z) amino - 2(S) propyl) - (S) Lys - (S) ABO - (S)Arg OH

En remplaçant dans l'exemple 5 le (2S, 3R)AHPA - (S) Lys (Z) - OH par le (Phényl - 3 (Z) - amino - 2(S) propyl) - (S) Lys (Z) - OH obtenu au stade précédent on obtient successivement :

* (Phényl - 3 (Z) - amino - 2(S) propyl) - (S) Lys (Z) - (S) ABO - (S) Arg ($NO_2$) $OCH_2$ - $C_6H_5$

* (Phényl - 3 amino - 2(S) propyl) - (S) Lys - (S) ABO - (S) Arg OH lyophilisé sous forme de triacétate

Caractéristiques spectrales :

Spectrométrie de masse :

Spectre $FAB^+$ $[M + H]^+$ M/Z : 573

Spectre $FAB^-$ $[M - H]^-$ M/Z : 571

**EXEMPLE 11 :**

(Hydroxy - 2(S) amino - 3(S) phényl - 4 butyl) - (S) Lys - (S) ABO - (S) Arg - OH (isomère α)
et

**EXEMPLE 12 :**

(Hydroxy - 2(R) amino - 3(S) phényl - 4 butyl) - (S) Lys - (S) ABO - (S) Arg - OH (isomère β)

**STADE A :** (S) Benzyloxycarbonylamino - 3 phényl - 4 oxo - 2 bromo - 1 butane

L'anhydride mixte entre la (Z) - (S) Phénylalanine et le chloroformiate d'isobutyle est préparé selon la technique décrite par H. BODANSZKY, A. BODANSZKY - ("The practice of peptide synthesis", p 109 Springer Verlag (1984)), on obtient ainsi une solution de 25 mmole de cet anhydride mixte dans 50 $cm^3$ de THF.

A cette solution refroidie à 0°C, ajouter, en 1 heure, une solution éthérée de diazométhane (1,8 g dans 250 $cm^3$), agiter 3h30 à 20°C puis chasser l'excès de diazométhane par un courant d'azote. La diazocétone obtenue (chromatographie couche mince : support silice Si 60F254, solvant toluène, acétate d'éthyle 75/25, Rf. : 0,25) est transformée en (S) Benzyloxycarbonylamino - 3 phényl - 4 oxo - 2 bromo - 1 butane selon la technique de IH. HALL, L.J. LOEFFLER (J. Med. Chem. 1980, 23, 275-278) avec un rendement de 68 %. Chromatographie couche mince : support silice Si 60F254, solvant toluène, acétate d'éthyle 75/25, Rf. : 0,45.

Caractéristiques spectrales :

νs NH : 3320 $cm^{-1}$

νs CO (carbamate) : 1740 $cm^{-1}$

νs CO (cétone) : 1690 $cm^{-1}$

**STADE B :** N [(S) Benzyloxycarbonylamino - 3 phényl - 4 oxo - 2 butyl] - (S) Lys (Z) OH

20 mmoles de (S) Benzyloxycarbonylamino - 3 phényl - 4 oxo - 2 bromo - 1 butane obtenues au stade précédent sont mises en solution dans un mélange de 90 $cm^3$ de tétrahydrofuranne et 10 $cm^3$ de diméthylformamide. Ajouter 2 mmoles de trifluoroacétate de (S) Lys (Z) OtBu (commercial) et 4 mmoles de carbonate de sodium puis porter 3 heures au reflux. Evaporer à sec et redissoudre dans 100 $cm^3$ d'acétate d'éthyle ; laver par une solution saturée de chlorure de sodium, sécher sur sulfate de magnésium et évaporer. Le N [(S) Benzyloxycarbonylamino - 3 phényl - 4 oxo - 2 butyl] - (S) Lys (Z) - OtBu est purifié par chromatographie sur gel de silice (solvant $CH_2Cl_2$ 95 MeOH 5) et dissous dans 50 $cm^3$ d'une solution 2N d'acide chlorhydrique dans l'acétate d'éthyle. Après 18 heures à 20°C, évaporer à secet concrétiser dans l'éther sous forme de chlorhydrate le N [(S) Benzyloxycarbonylamino - 3 phényl - 4 oxo - 2 butyl] - (S) Lys (Z) - OH qui est utilisé tel quel au stade suivant.

**STADE C :** N [(S) Benzyloxycarbonylamino - 3 phényl - 4 oxo - 2 butyl] - (S) Lys (Z) - (S) ABO - (S) Arg ($NO_2$) - $OCH_2C_6H_5$

En remplaçant dans l'exemple 5 le (2S, 3R)AHPA - (S) Lys (Z) - OH par le N [(S) Benzyloxycarbonylamino - 3 phényl - 4 oxo - 2 butyl] - (S) Lys (Z) - OH obtenu au stade précédent on obtient le N [(S) Benzyloxycarbonylamino - 3 phényl - 4 oxo - 2 butyl] - (S) Lys (Z) - (S) ABO - (S) Arg ($NO_2$) - $OCH_2C_6H_5$, purifié par chromatographie sur gel de silice. (solvant $CH_2Cl_2$, MeOH 90/10, Rf. : 0,65).

**STADE D :**

(Hydroxy - 2(S)(Z) - amino - 3 (S) phényl - 4 butyl) - (S) Lys (Z)- (S) ABO - (S) Arg ($NO_2$)- $OCH_2C_6H_5$ (isomère α)
et
(Hydroxy - 2(R)(Z) - amino - 3 (S) phényl - 4 butyl) - (S) Lys (Z)- (S) ABO - (S) Arg ($NO_2$)- $OCH_2C_6H_5$ (isomère β)

La fonction cétone du N [(S) Benzyloxycarbonylamino - 3 phényl - 4 oxo - 2 butyl] - (S) Lys (Z) - (S) ABO - (S) Arg ($NO_2$) - $OCH_2C_6H_5$ obtenu au stade précédent est réduite en alcool par action du borohydrure de sodium dans le méthanol. Les deux diastéréoisomères sont séparés par chromatographie sur gel de silice. (solvant : $CH_2Cl_2$ 90, Ethanol : 10).

**STADE E :**

(Hydroxy - 2(S) amino -3(S) phényl - 4 butyl) - (S)Lys - (S)ABO - (S)Arg - OH (isomère α)
et
(Hydroxy - 2(R) amino -3(S) phényl - 4 butyl) - (S)Lys - (S)ABO - (S)Arg - OH (isomère β)

Chacun des isomères obtenus au stade précédent est traité selon la technique utilisée dans l'exemple 1 au stade F pour donner :

**EXEMPLE 11 :** (Hydroxy - 2(S) amino -3(S) phényl - 4 butyl) - (S)Lys - (S) ABO - (S)Arg - OH (isomère α)

lyophilisé sous forme de triacétate.

**EXEMPLE 12 :** (Hydroxy - 2(R) - amino -3(S) phényl - 4 butyl) - (S)Lys - (S)ABO - (S)Arg - OH (isomère β)

lyophilisé sous forme de triacétate.

Spectrométrie de masse : (spectre DCI ($NH_3$))

Pour les deux isomères :

M/Z :

585 : $[M + H - H_2O]^+$

543 : $[M + H - H_2O - HN = . = NH]^+$

**EXEMPLE 13 ET 14 :**

(Hydroxy - 2(S) amino -3(R) phényl - 4 butyl) - (S)Lys - (S)ABO - (S)Arg - OH (isomère α)
et
(Hydroxy - 2(R) amino -3(R) phényl - 4 butyl) - (S)Lys - (S)ABO - (S)Arg - OH (isomère β)

En remplaçant dans le mode opératoire des exemples 11 et 12 la Z(S) Phénylalanine par la Z(R) Phénylalanine, on obtient successivement :

(R) Benzyloxycarbonylamino - 3 phényl - 4 oxo - 2 bromo - 1 butane

N [(R) Benzyloxycarbonylamino - 3 phényl - 4 oxo - 2 butyl] - (S)Lys(Z) - OH

N [(R) Benzyloxycarbonylamino - 3 phényl - 4 oxo - 2 butyl] - (S)Lys(Z) - (S)ABO - (S) Arg($NO_2$) $OCH_2$ $C_6H_5$

puis

(Hydroxy - 2(S) (Z) - amino - 3(R) phényl - 4 butyl) - (S)Lys(Z)- (S)ABO - (S)Arg($NO_2$) $OCH_2$ $C_6H_5$ (isomère α)

et

(Hydroxy - 2(R) (Z) amino - 3(R) phényl - 4 butyl) - (S)Lys - (S)ABO - (S)Arg($NO_2$) $OCH_2C_6H_5$ (isomère β)

puis

**EXEMPLE 13 :**

(Hydroxy - 2(S) - amino - 3(R) phényl - 4 butyl) - (S)Lys - (S)ABO - (S)Arg OH (isomère α) lyophilisé sous forme de triacétate.

**EXEMPLE 14 :**

(Hydroxy - 2(R) amino - 3(R) phényl - 4 butyl) - (S)Lys - (S)ABO - (S)Arg OH (isomère β) lyophilisé sous forme de triacétate

**EXEMPLES 15 à 24 :**

En opérant comme dans l'exemple 1 mais en remplaçant au stade A le tBoc(S)PHI OH par :
– la t butoxycarbonyl - 1 carboxy - 2 indoline ou tBoc(S)IND - OH (exemple 15)
– la t butoxycarbonyl - 2 carboxy - 1 isoindoline ou tBoc(S)ISI - OH (exemple 16)
– le t butoxycarbonyl - 2 aza - 2 carboxy - 3 bicyclo [2.2.1] heptane ou tBoc (S)ABH - OH (exemple 17)
– la t butoxycarbonyl - 2 carboxy - 3 tétrahydro - 1,2,3,4 bétacarboline ou tBoc - (S) THC - OH (exemple 18)
– le t butoxycarbonyl - 2 carboxy - 1 perhydroiisoindole ou tBoc - PHII - OH (exemple 19)
– la t butoxycarbonyl - 1 carboxy 2 perhydroquinoléine ou tBoc - PHQ - OH (exemple 20)
– la t butoxycarbonyl - 2 carboxy - 3 perhydroisoquinoléine ou tBoc - PHIQ - OH (exemple 21)
– le t butoxycarbonyl - 1 carboxy - 2 perhydrocyclopenta [b] pyrrole ou tBoc - PCP - OH (exemple 22)
– la t butoxycarbonyl - 1 carboxy - 2 tétrahydro - 1,2,3,4 quinoléine ou tBoc THQ - OH (exemple 23)
– la t butoxycarbonyl - 2 carboxy - 3 tétrahydro - 1,2,3,4 isoquinoléine ou tBoc (S)THIQ - OH (exemple 24),

on obtient respectivement :

**EXEMPLE 15 :**

(2S, 3R)AHPA - (S)Lys - (S)IND - (S)Arg - OH
lyophilisé sous forme de diacétate.

**EXEMPLE 16 :**

(2S, 3R)AHPA - (S)Lys - (S)ISI - (S)Arg - OH
Lyophilisé sous forme de diacétate

**EXEMPLE 17 :**

(2S, 3R)AHPA - (S)Lys - (S)ABH - (S)Arg - OH
Lyophilisé sous forme de diacétate

**EXEMPLE 18 :**

(2S, 3R)AHPA - (S)Lys - (S)THC - (S)Arg - OH
Lyophilisé sous forme de diacétate

**EXEMPLE 19 :**

(2S, 3R)AHPA - (S)Lys - PHII - (S)Arg - OH
Lyophilisé sous forme de diacétate

**EXEMPLE 20 :**

(2S, 3R)AHPA - (S)Lys - PHQ - (S)Arg - OH
Lyophilisé sous forme de diacétate

**EXEMPLE 21 :**

(2S, 3R)AHPA - (S)Lys - PHIQ - (S)Arg - OH
Lyophilisé sous forme de diacétate

**EXEMPLE 22 :**

(2S, 3R)AHPA - (S)Lys - PCP - (S)Arg - OH
Lyophilisé sous forme de diacétate

**EXEMPLE 23 :**

(2S, 3R)AHPA - (S)Lys - THQ - (S)Arg - OH
Lyophilisé sous forme de diacétate

**EXEMPLE 24 :**

(2S, 3R)AHPA - (S)Lys - (S)THIQ - (S)Arg - OH
Lyophilisé sous forme de diacétate

**ETUDE PHARMACOLOGIQUE DES COMPOSES DE L'INVENTION**

La capacité des composés de l'invention de stimuler l'activité des cellules immuno-compétentes a été vérifiée in vitro et in vivo.

**EXEMPLE 25 :** Stimulation de la phagocytose in vitro

In vitro, la technique décrite par DESCAMPS B., 1980 (Ann. Immunol. Inst. Past., 131 C, N° 2, p. 10) a été utilisée pour mesurer la stimulation des capacités phagocytaires de macrophages par les composés de l'invention : des macrophages péritonéaux de souris (souche $B_6D_2F_1$) sont ensemencés sur des boîtes de Pétri à raison de $10^4$ cellules par boite. Après attachement de la culture au milieu, les composés de l'invention sont ajoutés en solution aqueuse à une concentration 25 micromolaire par boite, puis des globules rouges de mouton opsonisés par des immunoglobulines spécifiques sont introduits. Après 1 heure de contact, les cultures sont lavées et l'on dénombre les macrophages ayant ingéré plus de 2 globules rouges.

Les composés de l'invention augmentent d'environ 25 % le pouvoir phagocytaire des macrophages par rapport à des cultures témoins. Dans les mêmes conditions, le pouvoir d'activation de la tuftsine est de 15 %.

**EXEMPLE 26 :** Promotion de l'activité N.K.

Les composés selon l'invention ont également été testés pour leur pouvoir promoteur de l'activité "Tueuse spontanée" ("Natural killer"). Les cellules dotées de ce pouvoir forment la première ligne de défense de l'organisme vis-à-vis de l'envahissement septique, viral ou tumoral.

Pour apprécier leur pouvoir stimulant, des composés selon l'invention ont été étudiés selon la technique de REYNOLDS, et coll. 1981, (J. Immunol. 127, 282).

Les composés sont injectés par voie intraveineuse à la dose de 20 à 50 µg/kg à des souris de souche $B_6D_2F_1$.

Trois jours après le traitement, les animaux sont sacrifiés, leur rate prélevée et dissociée en ses cellules constituantes qui sont ensemencées en culture en présence de cellules tumorales YAC-1 préalablement marquées au chrome radioactif. A l'issue de l'incubation, le pouvoir destructeur des composés de l'invention est mesuré par la quantité de chrome libérée.

Les composés de l'invention à une dose de 25 µg/kg, induisent une augmentation, par rapport au témoin, de la libération de chrome de l'ordre de 15 %, alors que la tuftsine à la dose de 40 µg/kg ne provoque qu'une libération de 10 %.

**EXEMPLE 27 :** Inhibition de croissance du mélanome B 16

Les mélanomes sont des tumeurs cancéreuses sensibles à la réaction du système immunitaire du malade. Ils constituent donc un modèle de choix pour apprécier toute stimulation de défense antitumorale.

Les composés de l'invention se sont montrés capables de ralentir de 40 % la croissance du mélanome B 16 de la souris, lorsque les produits sont administrés à raison de 20 μg/kg, 3 fois par semaine par voie intrapéritonéale. Dans les mêmes conditions, la tuftsine s'est montrée incapable de favoriser le ralentissement de croissance de la tumeur greffée.

**EXEMPLE 28 :** Augmentation de la résistance des animaux à l'infection

Certaines souches bactériennes pathogènes sont capables de tuer l'hôte sain chez qui elles sont inoculées. C'est le cas, par exemple, de Klebsiella pneumoniae, agent responsable de la pneumonie (Parant, M. ; et coll. Proc. Natl. Acad. Sci. USA, 1978, 75, n° 7, 3395).

Les composés de l'invention sont capables à la dose de 60μg par animal de protéger de la mort par infection toutes les souris Swiss, femelles, de 20 à 25 g., auxquelles la souche Klebsiella pneumoniae 7823 est inoculée par voie IP, lorsqu'ils sont administrés 48 heures avant l'infection. Dans les mêmes conditions, la tufstine n'a été capable de sauver que 20 % des animaux.

**EXEMPLE 29 :** Augmentation de la réponse lymphocytaire aux mitogènes

Des lectines extraites de plantes sont capables de se substituer in vitro à la stimulation de la prolifération des lymphocytes normalement assurée par des antigènes spécifiques. Ces agents sont des mitogènes lymphocytaires.

Après la mise en contact des lymphocytes avec un tel mitogène, on peut mesurer l'intensité de la prolifération et ainsi mesurer la réactivité immunoprotectrice des animaux traités par un composé (Daguillar, F. Med. Clin. North. Am., 56, 293).

Ainsi, des lymphocytes spléniques de souris traitées par 0,5 mg par kg des composés selon l'invention exposés à la Concanavaline A à une concentration de 0,5 mg/ml répondent par une prolifération environ égale à 1,8 fois celle de lymphocytes d'animaux non traités.

**EXEMPLE 30 :** Compositions pharmaceutiques

Soluté injectable

| | | | |
|---|---|---|---|
| (2S, 3R)AHPA - (S)Lys - (S)PHI - (S)Arg OH | | 0,020 | g |
| Eau pour préparation injectable | q.s.p. | 1,5 | $cm^3$ |

Comprimé

Formule de préparation pour 1000 comprimés :

| | | |
|---|---|---|
| (2S, 3R)AHPA - (S)Lys - (S)PHI - (S)Arg OH | 25 | g |
| Hydroxy propylcellulose | 1 | g |
| Amidon de blé | 10 | g |
| Lactose | 100 | g |
| Stéarate de magnésium | 2 | g |
| Talc | 2 | g |

## Revendications

**Revendications pour les Etats contractants suivants : AT, BE, CH, DE, FR, GB, IT, LI, LU, NL, SE**

1. Composés de formule générale :

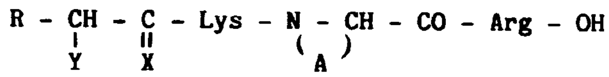

(I)

dans laquelle :
X représente :

- soit un atome d'oxygène,
- soit deux atomes d'hydrogène,

Y représente :

- soit un atome d'hydrogène,
- soit un groupement hydroxyle,

ou
Y représente un groupement amino à la condition que X représente deux atomes d'hydrogène ;
R représente un atome d'hydrogène, un groupement alcoyle de 1 à 6 atomes de carbone en chaîne droite ou ramifiée éventuellement substitué par un ou plusieurs groupements hydroxy, amino, mercapto, méthylthio, carboxy ou aryle tel que phényle, pyridyle ou thiényle ;
Lys et Arg représentent respectivement les restes lysyle et arginyle engagés dans des liaisons peptidiques,

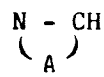

représente
* une structure bicyclique de formule :

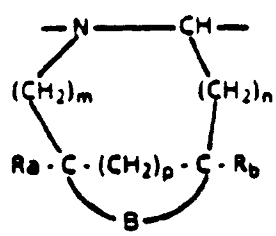

où

- m est égal à 1 ou zéro,
- n et p représentent zéro, 1 ou 2,
- Ra et Rb représentent un atome d'hydrogène ou peuvent former ensemble une liaison directe quand p = 0,
- B représente :
  * une chaîne alkylène $(CH_2)q$ où q est égal à 2, 3 ou 4,
  * ou une structure insaturée $( - CH = CH -)_2$ quand p = 0 et Ra et Rb forment ensemble une liaison, avec la réserve que la somme m, n, p et q est un nombre entier compris entre 3 et 6, ou
  * la tétrahydro - 1, 2, 3, 4 - bétacarboline. leurs énantiomères, épimères et diastéréoisomères,

ainsi que leurs sels d'addtion à un acide ou une base pharmaceutiquement acceptable.

2. Composés selon la revendication 1 dans lesquels la structure cyclique

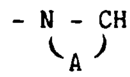

représente l'indoline, l'isoindoline, la tétrahydroquinoléine, la tétrahydroisoquinoléine, le perhydroindole, le perhydroisoindole, la perhydroquinoléine, la perhydroisoquinoléine, le perhydrocyclopenta [b] pyrrole, l'aza - 2 bicyclo [2.2.2] octane, l'aza - 2 bicyclo [2.2.1] heptane, la tétrahydro - 1, 2, 3, 4 bétacarboline,
leurs isomères, épimères et diastéréoisomères, ainsi que leurs sels d'addition à un acide ou une base pharmaceutiquement acceptable.

3. Composés selon l'une quelconque des revendications 1 ou 2 dans lesquels la structure cyclique

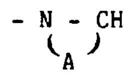

représente le perhydroindole ou l'azabicyclo [2.2.2] octane, leurs énantiomères, épimères et diastéréoisomères, ainsi que leurs sels d'addition à un acide ou une base pharmaceutiquement acceptable.

4. L'AHPA - (S)Lys - (S)PHI - (S)Arg - OH ses isomères et ses sels d'addition à un acide ou une base pharmaceutiquement acceptable.

5. Le (2S, 3R)AHPA - (S)Lys - (S)PHI - (S)Arg - OH et ses sels d'addition à un acide ou une base pharmaceutiquement acceptable.

6. L'AHPA - (S)Lys - (S)ABO - (S)Arg - OH, ses isomères et ses sels d'addition à un acide ou une base pharmaceutiquement acceptable.

7. Le N [hydroxy - 2 amino - 3(R) butyryl] - (S)Lys - (S)ABO - (S)Arg - OH, ses isomères et ses sels d'addition à un acide ou une base pharmaceutiquement acceptable.

8. Le (phényl - 3 amino - 2(S) propyl) - (S)Lys - (S)ABO - (S)Arg - OH, ses isomères et ses sels d'addition à un acide ou une base pharmaceutiquement acceptable.

9. L'(hydroxy - 2 amino - 3(S) phényl - 4 butyl) - (S)Lys - (S)ABO - (S)Arg - OH, ses isomères et ses sels d'addition à un acide ou une base pharmaceutiquement acceptable.

10. L'(hydroxy - 2 amino - 3(R) phényl - 4 butyl) - (S)Lys - (S)ABO - (S)Arg - OH, ses isomères et ses sels d'addition à un acide ou une base pharmaceutiquement acceptable.

11. Procédé de préparation des dérivés de formule (I) caractérisé en ce que l'on condense un dérivé de formule (II) :

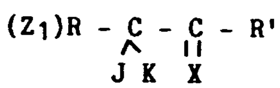 (II)

dans laquelle :

* R et X ont la même signification que dans la formule (I),
* R′ représente :
  – soit un atome d'hydrogène, soit un groupement hydroxyle lorsque X représente un atome d'oxygène,
  – soit un atome d'halogène et préférentiellement un atome de brome à la condition que dans ce cas X représente deux atomes d'hydrogène,
* J représente un atome d'hydrogène ou un groupement hydroxyle à la condition que K représente un atome d'hydrogène, ou bien
J représente un groupement benzyloxycarbonylamino à la double condition que K et R′ représentent chacun un atome d'hydrogène, ou bien
J et K représentent ensemble un atome d'oxygène à la condition que X représente deux atomes d'hydrogène,
* $(Z_1)$ représente un groupement protecteur des éventuels substituants aminés du radical R et en particulier le groupe benzyloxycarbonyle, avec un dérivé de formule (III) :

**Lys (Z) - OtBu (III)**

dans laquelle :
(Z) représente un groupement protecteur du substituant ω aminé et en particulier le groupe benzyloxycarbonyle, et tBu représente un groupement protecteur du substituant carboxyle et en particulier le radical tertiobutyle
en présence :

– d'un agent réducteur comme par exemple un hydrure mixte de métal alcalin, comme par exemple un borohydrure de métal alcalin ou bien un cyanoborohydrure de métal alcalin lorsque R′ représente un atome d'hydrogène,

– d'un agent de couplage peptidique usuel, tel que la dicyclohexylcarbodiimide en présence d'hydroxybenzotriazole, lorsque R′ représente un groupement hydroxyle,

– d'un agent basique comme par exemple un sel de métal alcalin, comme par exemple un carbonate de métal alcalin tel que le carbonate de sodium lorsque R′ représente un atome d'halogène,

pour obtenir un dérivé de formule (IV) :

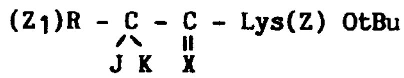

(IV)

dans laquelle R et X ont la même signification que dans la formule (I), J, K et ($Z_1$) la même signification que dans la formule (II) et (Z) et tBu la même signification que dans la formule (III), que l'on déprotège en milieu acide au niveau de la fonction acide carboxylique de la lysine en un dérivé de formule (V) :

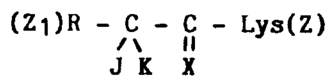

(V)

dans laquelle R et X ont la même signification que dans la formule (I), J, K et ($Z_1$) la même signification que dans la formule (II) et (Z) la même signification que dans la formule (III), qui est ensuite condensé avec un dérivé de formule (VI) :

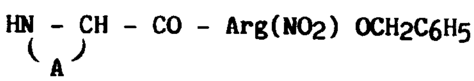

(VI)

dans laquelle A représente avec les atomes de carbone et d'azote auxquels il est attaché la même signification que dans la formule (I), lui même obtenu par condensation d'un dérivé de formule (VII) :

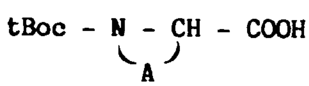

(VII)

dans laquelle A représente avec les atomes de carbone et d'azote auxquels il est attaché la même signification que dans la formule (I) et tBoc représente le radical tertiobutoxycarbonyle, avec le Nω - nitroarginate de benzyle (H - Arg($NO_2$) $OCH_2C_6H_5$) pour obtenir un dérivé de formule (VIII) :

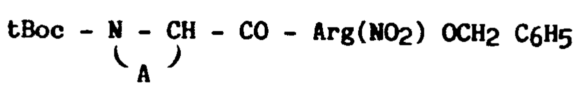

(VIII)

dans lequel A, avec les atomes de carbone et d'azote auxquels il est attaché, a la même signification que dans la formule (I), que l'on déprotège ensuite par l'acide trifluoroacétique en un dérivé de formule (VI), la condensation du dérivé de formule (V) avec le dérivé de formule (VI) permettant l'obtention d'un dérivé de formule (IX) :

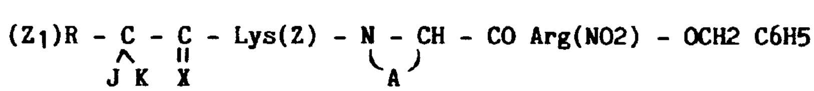

(IX)

dans lequel A avec les atomes de carbone et d'azote auquels il est attaché, R et X ont la même signification que dans la formule (I) J, K et ($Z_1$) la même signification que dans la formule (II) et (Z) la même signification que dans la formule (III),

qui est, lorsque J et K représentent ensemble un atome d'oxygène, soumis à l'action d'un hydrure mixte de métal alcalin tel que le borohydrure de sodium, le composé ainsi obtenu pouvant être, si on le désire, séparé en ses isomères par une technique classique de séparation comme la chromatographie sur colonne de silice,

pour obtenir un composé de formule générale (IXa) cas particulier des dérivés de formule (IX) dans laquelle A avec les atomes de carbone et d'azote auxquels il est attaché, R, X, ($Z_1$), et (Z) ont la même signification que dans la formule (IX), J représente un groupement hydroxyle et K représente un atome d'hydrogène,

dérivé de formule (IX) ou (IXa) que l'on déprotège par hydrogénolyse dans un solvant polaire acide en présence d'un catalyseur d'hydrogénation,

pour conduire à un dérivé de formule (I) que l'on peut, si on le désire :

- soit salifier par un acide ou une base pharmaceutiquement acceptable,
- soit séparer en ses isomères, puis, si nécessaire, salifier par un acide ou une base pharmaceutiquement acceptable.

12. Composés de structure générale (IX) et (IXa) selon la revendication 11 utiles pour la préparation des composés selon la revendication 1.

13. Composition pharmaceutique contenant comme principe actif au moins un composé selon l'une des revendications 1 à 10 en combinaison avec un ou plusieurs excipients ou véhicules inertes non-toxiques, pharmaceutiquement acceptables.

14. Composition pharmaceutique selon la revendication 13 utile dans le traitement des affections liées à une perturbation des défenses immunitaires de l'organisme et notamment dans le traitement des cancers, affections virales, bactériennes ou fongiques, des maladies auto immunes comme le lupus érythémateux ou l'arthrite rhumatoide.

**Revendications pour l'Etat contractant suivant: GR**

1. Procédé de préparation des composés de formule générale (I) :

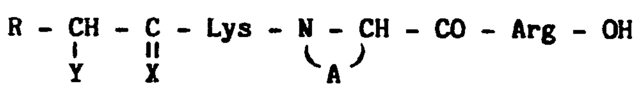

(I)

dans laquelle :

X représente :

- soit un atome d'oxygène,
- soit deux atomes d'hydrogène,

Y représente :

- soit un atome d'hydrogène,
- soit un groupement hydroxyle,

ou

Y représente un groupement amino à la condition que X représente deux atomes d'hydrogène ;

R représente un atome d'hydrogène, un groupement alcoyle de 1 à 6 atomes de carbone en chaîne droite ou ramifiée éventuellement substitué par un ou plusieurs groupements hydroxy, amino, mercapto, méthylthio, carboxy ou aryle tel que phényle, pyridyle ou thiényle ;

Lys et Arg représentent respectivement les restes lysyle et arginyle engagés dans des liaisons peptidiques,

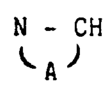

représente

* une structure bicyclique de formule :

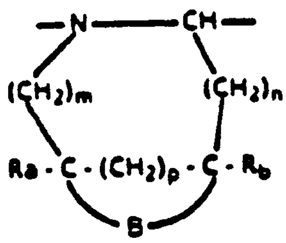

où

- m est égal à 1 ou zéro,
- n et p représentent zéro, 1 ou 2,
- Ra et Rb représentent un atome d'hydrogène ou peuvent former ensemble une liaison directe quand p = 0,
- B représente :
  * une chaîne alkylène $(CH_2)q$ où q est égal à 2, 3 ou 4,
  * ou une structure insaturée $( - CH = CH -)_2$ quand p = 0 et Ra et Rb forment ensemble une liaison, avec la réserve que la somme m, n, p et q est un nombre entier compris entre 3 et 6, ou
  * la tétrahydro - 1, 2, 3, 4 - bétacarboline.

leurs énantiomères, épimères et diastéréoisomères,
ainsi que leurs sels d'addition à un acide ou une base pharmaceutiquement acceptable, caractérisé en ce que l'on condense un dérivé de formule (II) :

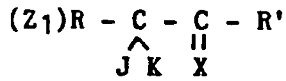

(II)

dans laquelle :

* R et X ont la même signification que dans la formule (I),
* R′ représente :
  - soit un atome d'hydrogène, soit un groupement hydroxyle lorsque X représente un atome d'oxygène,
  - soit un atome d'halogène et préférentiellement un atome de brome à la condition que dans ce cas X représente deux atomes d'hydrogène,
* J représente un atome d'hydrogène ou un groupement hydroxyle à la condition que K représente un atome d'hydrogène, ou bien
  J représente un groupement benzyloxycarbonylamino à la double condition que K et R′ représentent chacun un atome d'hydrogène, ou bien
  J et K représentent ensemble un atome d'oxygène à la condition que X représente deux atomes d'hydrogène,
* $(Z_1)$ représente un groupement protecteur des éventuels substituants aminés du radical R et en particulier le groupe benzyloxycarbonyle,

avec un dérivé de formule (III) :

**Lys (Z) - OtBu (III)**

dans laquelle :
(Z) représente un groupement protecteur du substituant ω aminé et en particulier le groupe benzyloxycarbonyle, et tBu représente un groupement protecteur du substituant carboxyle et en particulier le radical tertiobutyle en présence :

- d'un agent réducteur comme par exemple un hydrure mixte de métal alcalin, comme par exemple un borohydrure de métal alcalin ou bien un cyanoborohydrure de métal alcalin lorsque R′ représente un atome d'hydrogène,
- d'un agent de couplage peptidique usuel, tel que la dicyclohexylcarbodiimide en présence d'hydroxybenzotriazole, lorsque R′ représente un groupement hydroxyle,
- d'un agent basique comme par exemple un sel de métal alcalin, comme par exemple un carbonate de métal alcalin tel que le carbonate de sodium lorsque R′ représente un atome d'halogène,

pour obtenir un dérivé de formule (IV) :

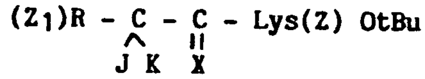

(IV)

dans laquelle R et X ont la même signification que dans la formule (I), J, K et ($Z_1$) la même signification que dans la formule (II) et (Z) et tBu la même signification que dans la formule (III), que l'on déprotège en milieu acide au niveau de la fonction acide carboxylique de la lysine en un dérivé de formule (V) :

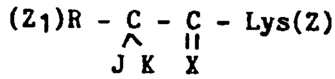

(V)

dans laquelle R et X ont la même signification que dans la formule (I), J, K et ($Z_1$) la même signification que dans la formule (II) et (Z) la même signification que dans la formule (III), qui est ensuite condensé avec un dérivé de formule (VI) :

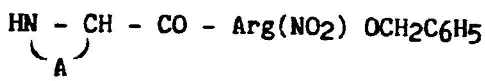

(VI)

dans laquelle A représente avec les atomes de carbone et d'azote auxquels il est attaché la même signification que dans la formule (I), lui même obtenu par condensation d'un dérivé de formule (VII) :

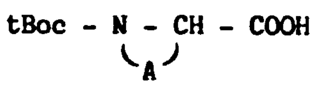

(VII)

dans laquelle A représente avec les atomes de carbone et d'azote auxquels il est attaché la même signification que dans la formule (I) et tBoc représente le radical tertiobutoxycarbonyle, avec le Nω - nitroarginate de benzyle (H - Arg($NO_2$) $OCH_2C_6H_5$) pour obtenir un dérivé de formule (VIII) :

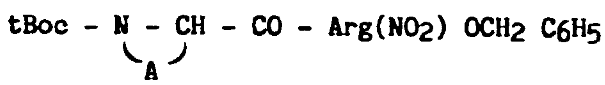

(VIII)

dans lequel A, avec les atomes de carbone et d'azote auxquels il est attaché, a la même signification que dans la formule (I), que l'on déprotège ensuite par l'acide trifluoroacétique en un dérivé de formule (VI), la condensation du dérivé de formule (V) avec le dérivé de formule (VI) permettant l'obtention d'un dérivé de formule (IX) :

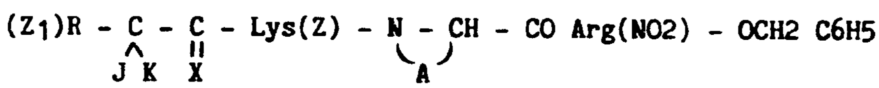

(IX)

dans lequel A avec les atomes de carbone et d'azote auqxuels il est attaché, R et X ont la même signification que dans la formule (I) J, K et ($Z_1$) la même signification que dans la formule (II) et (Z) la même signification que dans la formule (III), qui est, lorsque J et K représentent ensemble un atome d'oxygène, soumis à l'action d'un hydrure mixte de métal alcalin tel que le borohydrure de sodium, le composé ainsi obtenu pouvant être, si on le désire, séparé en ses isomères par une technique classique de séparation comme la chromatographie sur colonne de silice, pour obtenir un composé de formule générale (IXa) cas particulier des dérivés de formule (IX) dans laquelle A avec les atomes de carbone et d'azote auxquels il est attaché, R, X, ($Z_1$), et (Z) ont la même signification que dans la formule (IX), J représente un groupement hydroxyle et K représente un atome d'hydrogène,

dérivé de formule (IX) ou (IXa) que l'on déprotège par hydrogénolyse dans un solvant polaire acide en présence d'un catalyseur d'hydrogénation,
pour conduire à un dérivé de formule (I) que l'on peut, si on le désire :

– soit salifier par un acide ou une base pharmaceutiquement acceptable,
– soit séparer en ses isomères, puis, si nécessaire, salifier par un acide ou une base pharmaceutiquement acceptable.

2. Procédé selon la revendication 1 de préparation de composés dans lesquels la structure cyclique

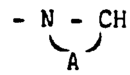

représente l'indoline, l'isoindoline, la tétrahydroquinoléine, la tétrahydroisoquinoléine, le perhydroindole, le perhydroisoindole, la perhydroquinoléine, la perhydroisoquinoléine, le perhydrocyclopenta [b] pyrrole, l'aza - 2 bicyclo [2.2.2] octane, l'aza - 2 bicyclo [2.2.1] heptane, la tétrahydro - 1, 2, 3, 4 bétacarboline,
leurs isomères, épimères et diastéréoisomères, ainsi que leurs sels d'addition à un acide ou une base pharmaceutiquement acceptable.

3. Procédé selon l'une quelconque des revendications 1 ou 2 de préparation des composés dans lesquels la structure cyclique

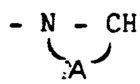

représente le perhydroindole ou l'azabicyclo [2.2.2] octane, leurs énantiomères, épimères et diastéréoisomères, ainsi que leurs sels d'addition à un acide ou une base pharmaceutiquement acceptable.

4. Procédé selon l'une des revendications 1 à 3 de préparation de l'AHPA - (S)Lys - (S)PHI - (S)Arg - OH ses isomères et ses sels d'addition à un acide ou une base pharmaceutiquement acceptable.

5. Procédé selon l'une des revendications 1 à 3 de préparation du (2S, 3R)AHPA - (S)Lys - (S)PHI - (S)Arg - OH et ses sels d'addition à un acide ou une base pharmaceutiquement acceptable.

6. Procédé selon l'une des revendications 1 à 3 de préparation de l'AHPA - (S)Lys - (S)ABO - (S)Arg - OH, ses isomères et ses sels d'addition à un acide ou une base pharmaceutiquement acceptable.

7. Procédé selon l'une des revendications 1 à 3 de préparation du N [hydroxy - 2 amino - 3(R) butyryl] - (S)Lys - (S)ABO - (S)Arg - OH, ses isomères et ses sels d'addition à un acide ou une base pharmaceutiquement acceptable.

8. Procédé selon l'une des revendications 1 à 3 de préparation du (phényl - 3 amino - 2(S) propyl) - (S)Lys - (S)ABO - (S)Arg - OH, ses sels d'addition à un acide ou une base pharmaceutiquement acceptable.

9. Procédé selon l'une des revendications 1 à 3 de préparation de l'(hydroxy - 2 amino - 3(S) phényl - 4 butyl) - (S)Lys - (S)ABO - (S)Arg - OH, ses isomères et ses sels d'addition à un acide ou une base pharmaceutiquement acceptable.

10. Procédé selon l'une des revendications 1 à 3 de préparation de l'(hydroxy - 2 amino - 3(R) phényl - 4 butyl) - (S)Lys - (S)ABO - (S)Arg - OH, ses isomères et ses sels d'addition à un acide ou une base pharmaceutiquement acceptable.

11. Procédé de préparation d'une composition pharmaceutique contenant comme principe actif au moins un composé selon l'une des revendications 1 à 10 en combinaison avec un ou plusieurs excipients ou véhicules inertes non-toxiques, pharmaceutiquement acceptables.

**Revendications pour l'Etat contractant suivant: ES**

1. Procédé de préparation des composés de formule générale (I) :

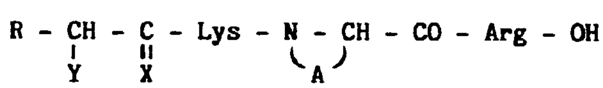 (I)

dans laquelle :
X représente :

– soit un atome d'oxygène,

– soit deux atomes d'hydrogène,

Y représente :

– soit un atome d'hydrogène,

– soit un groupement hydroxyle,

ou

Y représente un groupement amino à la condition que X représente deux atomes d'hydrogène ;

R représente un atome d'hydrogène, un groupement alcoyle de 1 à 6 atomes de carbone en chaîne droite ou ramifiée éventuellement substitué par un ou plusieurs groupements hydroxy, amino, mercapto, méthylthio, carboxy ou aryle tel que phényle, pyridyle ou thiényle ;

Lys et Arg représentent respectivement les restes lysyle et arginyle engagés dans des liaisons peptidiques,

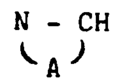

représente

* une structure bicyclique de formule :

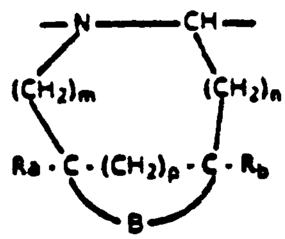

où

– m est égal à 1 ou zéro,

– n et p représentent zéro, 1 ou 2,

– Ra et Rb représentent un atome d'hydrogène ou peuvent former ensemble une liaison directe quand p = 0,

– B représente :

* une chaîne alkylène $(CH_2)q$ où q est égal à 2, 3 ou 4,

* ou une structure insaturée $( - CH = CH -)_2$ quand p = 0 et Ra et Rb forment ensemble une liaison, avec la réserve que la somme m, n, p et q est un nombre entier compris entre 3 et 6, ou

* la tétrahydro - 1, 2, 3, 4 - bétacarboline.

leurs énantiomères, épimères et diastéréoisomères,

ainsi que leurs sels d'addtion à un acide ou une base pharmaceutiquement acceptable, caractérisé en ce que l'on condense un dérivé de formule (II) :

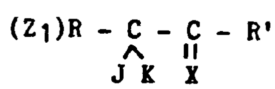

(II)

dans laquelle :

* R et X ont la même signification que dans la formule (I),

* R' représente :

– soit un atome d'hydrogène, soit un groupement hydroxyle lorsque X représente un atome d'oxygène,

– soit un atome d'halogène et préférentiellement un atome de brome à la condition que dans ce cas X représente deux atomes d'hydrogène,

* J représente un atome d'hydrogène ou un groupement hydroxyle à la condition que K représente un atome d'hydrogène, ou bien

J représente un groupement benzyloxycarbonylamino à la double condition que K et R' représentent chacun un atome d'hydrogène, ou bien

J et K représentent ensemble un atome d'oxygène à la condition que X représente deux atomes d'hydrogène,

* $(Z_1)$ représente un groupement protecteur des éventuels substituants aminés du radical R et en particulier le groupe benzyloxycarbonyle,

avec un dérivé de formule (III) :

**Lys (Z) - OtBu (III)**

dans laquelle :

(Z) représente un groupement protecteur du substituant ω aminé et en particulier le groupe benzyloxycarbonyle, et tBu représente un groupement protecteur du substituant carboxyle et en particulier le radical tertiobutyle en présence :

– d'un agent réducteur comme par exemple un hydrure mixte de métal alcalin, comme par exemple un borohydrure de métal alcalin ou bien un cyanoborohydrure de métal alcalin lorsque R′ représente un atome d'hydrogène,

– d'un agent de couplage peptidique usuel, tel que la dicyclohexylcarbodiimide en présence d'hydroxybenzotriazole, lorsque R′ représente un groupement hydroxyle,

– d'un agent basique comme par exemple un sel de métal alcalin, comme par exemple un carbonate de métal alcalin tel que le carbonate de sodium lorsque R′ représente un atome d'halogène,

pour obtenir un dérivé de formule (IV) :

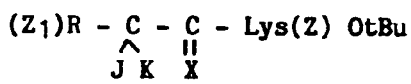

(IV)

dans laquelle R et X ont la même signification que dans la formule (I), J, K et $(Z_1)$ la même signification que dans la formule (II) et (Z) et tBu la même signification que dans la formule (III), que l'on déprotège en milieu acide au niveau de la fonction acide carboxylique de la lysine en un dérivé de formule (V) :

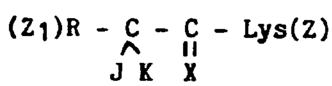

(V)

dans laquelle R et X ont la même signification que dans la formule (I), J, K et $(Z_1)$ la même signification que dans la formule (II) et (Z) la même signification que dans la formule (III), qui est ensuite condensé avec un dérivé de formule (VI) :

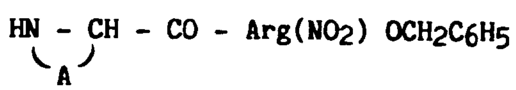

(VI)

dans laquelle A représente avec les atomes de carbone et d'azote auxquels il est attaché la même signification que dans la formule (I),

lui même obtenu par condensation d'un dérivé de formule (VII) :

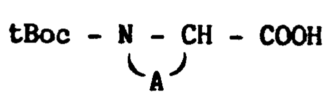

(VII)

dans laquelle A représente avec les atomes de carbone et d'azote auxquels il est attaché la même signification que dans la formule (I) et tBoc représente le radical tertiobutoxycarbonyle,

avec le Nω - nitroarginate de benzyle (H - $Arg(NO_2)$ $OCH_2C_6H_5$) pour obtenir un dérivé de formule (VIII) :

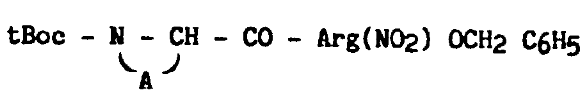

(VIII)

dans lequel A, avec les atomes de carbone et d'azote auxquels il est attaché, a la même signification que dans

la formule (I),
que l'on déprotège ensuite par l'acide trifluoroacétique en un dérivé de formule (VI),
la condensation du dérivé de formule (V) avec le dérivé de formule (VI) permettant l'obtention d'un dérivé de formule (IX) :

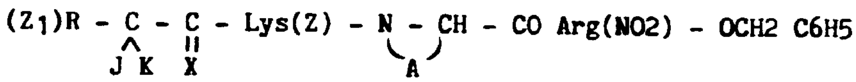 (IX)

dans lequel A avec les atomes de carbone et d'azote auqxuels il est attaché, R et X ont la même signification que dans la formule (I) J, K et ($Z_1$) la même signification que dans la formule (II) et (Z) la même signification que dans la formule (III),
qui est, lorsque J et K représentent ensemble un atome d'oxygène, soumis à l'action d'un hydrure mixte de métal alcalin tel que le borohydrure de sodium, le composé ainsi obtenu pouvant être, si on le désire, séparé en ses isomères par une technique classique de séparation comme la chromatographie sur colonne de silice, pour obtenir un composé de formule générale (IXa) cas particulier des dérivés de formule (IX) dans laquelle A avec les atomes de carbone et d'azote auxquels il est attaché, R, X, ($Z_1$), et (Z) ont la même signification que dans la formule (IX), J représente un groupement hydroxyle et K représente un atome d'hydrogène,
dérivé de formule (IX) ou (IXa) que l'on déprotège par hydrogénolyse dans un solvant polaire acide en présence d'un catalyseur d'hydrogénation,
pour conduire à un dérivé de formule (I) que l'on peut, si on le désire :

– soit salifier par un acide ou une base pharmaceutiquement acceptable,
– soit séparer en ses isomères, puis, si nécessaire, salifier par un acide ou une base pharmaceutiquement acceptable.

2. Procédé selon la revendication 1 de préparation de composés dans lesquels la structure cyclique

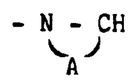

représente l'indoline, l'isoindoline, la tétrahydroquinoléine, la tétrahydroisoquinoléine, le perhydroindole, le perhydroisoindole, la perhydroquinoléine, la perhydroisoquinoléine, le perhydrocyclopenta [b] pyrrole, l'aza - 2 bicyclo [2.2.2] octane, l'aza - 2 bicyclo [2.2.1] heptane, la tétrahydro - 1, 2, 3, 4 bétacarboline,
leurs isomères, épimères et diastéréoisomères, ainsi que leurs sels d'addition à un acide ou une base pharmaceutiquement acceptable.

3. Procédé selon l'une quelconque des revendications 1 ou 2 de préparation des composés dans lesquels la structure cyclique

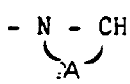

représente le perhydroindole ou l'azabicyclo [2.2.2] octane, leurs énantiomères, épimères et diastéréoisomères, ainsi que leurs sels d'addition à un acide ou une base pharmaceutiquement acceptable.

4. Procédé selon l'une des revendications 1 à 3 de préparation de l'AHPA - (S)Lys - (S)PHI - (S)Arg - OH ses isomères et ses sels d'addition à un acide ou une base pharmaceutiquement acceptable.

5. Procédé selon l'une des revendications 1 à 3 de préparation du (2S, 3R)AHPA - (S)Lys - (S)PHI - (S)Arg - OH et ses sels d'addition à un acide ou une base pharmaceutiquement acceptable.

6. Procédé selon l'une des revendications 1 à 3 de préparation de l'AHPA - (S)Lys - (S)ABO - (S)Arg - OH, ses isomères et ses sels d'addition à un acide ou une base pharmaceutiquement acceptable.

7. Procédé selon l'une des revendications 1 à 3 de préparation du N [hydroxy - 2 amino - 3(R) butyryl] - (S)Lys - (S)ABO - (S)Arg - OH, ses isomères et ses sels d'addition à un acide ou une base pharmaceutiquement acceptable.

8. Procédé selon l'une des revendications 1 à 3 de préparation du (phényl - 3 amino - 2(S) propyl) - (S)Lys - (S)ABO - (S)Arg - OH, ses sels d'addition à un acide ou une base pharmaceutiquement acceptable.

9. Procédé selon l'une des revendications 1 à 3 de préparation de l'(hydroxy - 2 amino - 3(S) phényl - 4 butyl) - (S)Lys - (S)ABO - (S)Arg - OH, ses isomères et ses sels d'addition à un acide ou une base pharmaceutiquement acceptable.

10. Procédé selon l'une des revendications 1 à 3 de préparation de l'(hydroxy - 2 amino - 3(R) phényl - 4 butyl) - (S)Lys - (S)ABO - (S)Arg - OH, ses isomères et ses sels d'addition à un acide ou une base pharmaceutiquement acceptable.

11. Procédé de préparation d'une composition pharmaceutique contenant comme principe actif au moins un composé selon l'une des revendications 1 à 10 en combinaison avec un ou plusieurs excipients ou véhicules inertes non-toxiques, pharmaceutiquement acceptables.

## Claims

### Claims for the following Contracting States: AT, BE, CH, DE, FR, GB, IT, LI, LU, NL, SE

1. Compounds of the general formula:

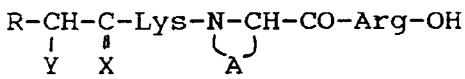 (I)

in which:
X represents:
- either an oxygen atom,
- or two hydrogen atoms,

Y represents:
- either a hydrogen atom,
- or a hydroxy group,

or
Y represents an amino group provided that X represents two hydrogen atoms;
R represents a hydrogen atom, a straight-chained or branched-chained alkyl group having from 1 to 6 carbon atoms that is optionally substituted by one or more hydroxy, amino, mercapto, methylthio, carboxy or aryl, such as phenyl, pyridyl or thienyl, groups;
Lys and Arg represent respectively peptide bonded lysyl and arginyl residues,

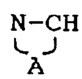

represents
* a bicyclic structure of the formula:

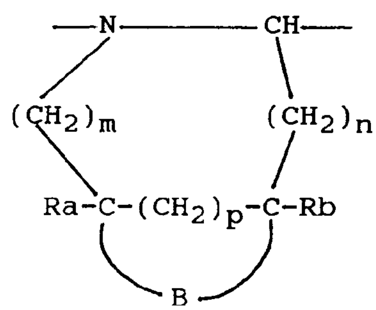

wherein
- m is 1 or 0,
- n and p represent 0, 1 or 2,
- Ra and Rb each represent a hydrogen atom or together can form a direct bond when p = 0,
- B represents:
  * an alkylene chain $(CH_2)q$ where q is 2, 3 or 4,
  * or an unsaturated structure $(-CH=CH-)_2$ when p = 0 and Ra and Rb together form a bond, with the proviso that the sum of m, n, p and q is an integer from 3 to 6, or

* 1,2,3,4-tetrahydrobetacarboline,

their enantiomers, epimers and diastereoisomers,
and also their addition salts with a pharmaceutically acceptable acid or base.

2. Compounds according to claim 1 in which the cyclic structure

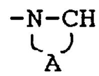

represents indoline, isoindoline, tetrahydroquinoline, tetrahydroisoquinoline, perhydroindole, perhydroisoindole, perhydroquinoline, perhydroisoquinoline, perhydrocyclopenta[b]pyrrole, 2-azabicyclo[2.2.2]octane, 2-azabicyclo[2.2.1]heptane, or 1,2,3,4-tetrahydrobetacarboline,
their isomers, epimers and diastereoisomers, and also their addition salts with a pharmaceutically acceptable acid or base.

3. Compounds according to either claim 1 or claim 2 in which the cyclic structure

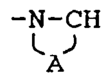

represents perhydroindole or azabicyclo[2.2.2]octane, their enantiomers, epimers and diastereoisomers, and also their addition salts with a pharmaceutically acceptable acid or base.

4. AHPA-(S)Lys-(S)PHI-(S)Arg-OH, its isomers and its addition salts with a pharmaceutically acceptable acid or base.

5. (2S,3R)AHPA-(S)Lys-(S)PHI-(S)Arg-OH and its addition salts with a pharmaceutically acceptable acid or base.

6. AHPA-(S)Lys-(S)ABO-(S)Arg-OH, its isomers and its addition salts with a pharmaceutically acceptable acid or base.

7. N-[2-hydroxy-3(R)-aminobutyryl]-(S)Lys-(S)ABO-(S)Arg-OH, its isomers and its addition salts with a pharmaceutically acceptable acid or base.

8. (3-phenyl-2(S)-aminopropyl)-(S)Lys-(S)ABO-(S)Arg-OH, its isomers and its addition salts with a pharmaceutically acceptable acid or base.

9. (2-hydroxy-3(S)-amino-4-phenylbutyl)-(S)Lys-(S)ABO-(S)Arg-OH, its isomers and its addition salts with a pharmaceutically acceptable acid or base.

10. (2-hydroxy-3(R)-amino-4-phenylbutyl)-(S)Lys-(S)ABO-(S)Arg-OH, its isomers and its addition salts with a pharmaceutically acceptable acid or base.

11. Process for the preparation of compounds of formula (I), characterised in that a compound of formula (II):

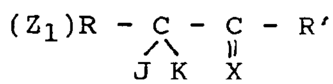

(II),

in which:

* R and X have the same meaning as in formula (I),
* R′ represents:
  - either a hydrogen atom or a hydroxy group when X represents an oxygen atom,
  - or a halogen atom and preferably a bromine atom, provided that in this case X represents two hydrogen atoms,
* J represents a hydrogen atom or a hydroxy group provided that K represents a hydrogen atom, or J represents a benzyloxycarbonylamino group provided that both K and R′ represent hydrogen atoms, or J and K together represent an oxygen atom provided that X represents two hydrogen atoms,
* ($Z_1$) represents a group protecting any amino substituents of the radical R that may be present, especially the benzyloxycarbonyl group,

is condensed with a compound of formula (III):

Lys(Z)-OtBu (III),

in which:
(Z) represents a group protecting the ω-amino substituent, especially the benzyloxycarbonyl group, and tBu

represents a group protecting the carboxy substituent, especially the tert.-butyl radical,
in the presence:

– of a reducing agent, such as, for example, an alkali metal mixed hydride, such as, for example, an alkali metal borohydride or an alkali metal cyanoborohydride, when R′ represents a hydrogen atom,
– of a customary peptide coupling agent, such as dicyclohexylcarbodiimide in the presence of hydroxybenzotriazole, when R′ represents a hydroxy group, or
– of a basic agent, such as, for example, an alkali metal salt, such as, for example, an alkali metal carbonate, such as sodium carbonate, when R′ represents a halogen atom, to obtain a compound of formula (IV):

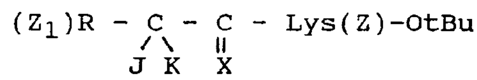

(IV)

in which R and X have the same meaning as in formula (I), J, K and ($Z_1$) have the same meaning as in formula (II), and (Z) and tBu have the same meaning as in formula (III),
which compound (IV) is deprotected in an acidic medium at the carboxylic acid function of the lysine to form a compound of formula (V):

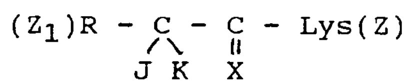

(V)

in which R and X have the same meaning as in formula (I), J, K and ($Z_1$) have the same meaning as in formula (II), and (Z) has the same meaning as in formula (III),
which compound (V) is then condensed with a compound of formula (VI):

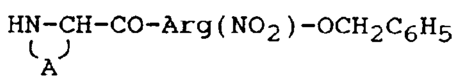

(VI),

in which A, together with the carbon and nitrogen atoms to which it is bonded, has the same meaning as in formula (I),
which is itself obtained by condensation of a compound of formula (VII):

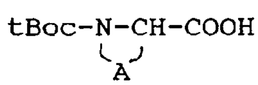

(VII),

in which A, together with the carbon and nitrogen atoms to which it is bonded, has the same meaning as in formula (I) and tBoc represents the tert.-butoxycarbonyl radical,
with benzyl $N_\omega$-nitroarginate (H-Arg($NO_2$)-$OCH_2C_6H_5$) to obtain a compound of formula (VIII):

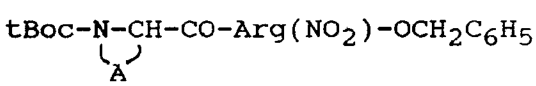

(VIII),

in which A, together with the carbon and nitrogen atoms to which it is bonded, has the same meaning as in formula (I),
which compound (VIII) is then deprotected by means of trifluoroacetic acid to form a compound of formula (VI), it being possible by condensation of the compound of formula (V) with the compound of formula (VI) to obtain a compound of formula (IX):

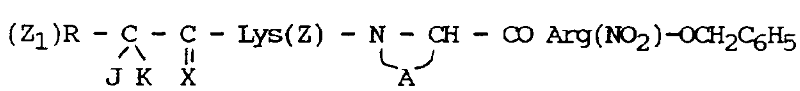

(IX),

in which A, together with the carbon and nitrogen atoms to which it is bonded, R and X have the same meaning

as in formula (I), J, K and ($Z_1$) have the same meaning as in formula (II), and (Z) has the same meaning as in formula (III),
which compound (IX), when J and K together represent an oxygen atom, is subjected to the action of an alkali metal mixed hydride, such as sodium borohydride, it being possible, if desired, to separate the resulting compound into its isomers by a conventional separation method, such as chromatography on a silica column,
to obtain a compound of the general formula (IXa), which is a special case of the compounds of formula (IX), in which A, together with the carbon and nitrogen atoms to which it is bonded, R, X, ($Z_1$) and (Z) have the same meaning as in formula (IX), J represents a hydroxy group and K represents a hydrogen atom,
which compound of formula (IX) or (IXa) is deprotected by hydrogenolysis in an acidic polar solvent in the presence of a hydrogenation catalyst,
to give a compound of formula (I) which may, if desired:

- either be converted into a salt by means of a pharmaceutically acceptable acid or base,
- or be separated into its isomers and then, if necessary, converted into a salt by means of a pharmaceutically acceptable acid or base.

12. Compounds of the general structure (IX) and (IXa) according to claim 11 that can be used for the preparation of the compounds according to claim 1.

13. Pharmaceutical composition containing as active ingredient at least one compound according to one of claims 1 to 10 in combination with one or more pharmaceutically acceptable inert, non-toxic excipients or vehicles.

14. Pharmaceutical composition according to claim 13 that can be used in the treatment of disorders associated with a disturbance in the immune defences of the organism and especially in the treatment of cancers, viral, bacterial or fungal disorders, autoimmune disorders, such as lupus erythematosus, or rheumatoid arthritis.

**Claims for the following Contracting State: GR**

1. Process for the preparation of compounds of the general formula (I):

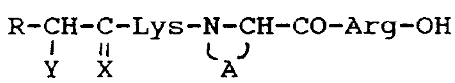

(I)

in which:
X represents:

- either an oxygen atom,
- or two hydrogen atoms,

Y represents:

- either a hydrogen atom,
- or a hydroxy group,

or
Y represents an amino group provided that X represents two hydrogen atoms;
R represents a hydrogen atom, a straight-chained or branched-chained alkyl group having from 1 to 6 carbon atoms that is optionally substituted by one or more hydroxy, amino, mercapto, methylthio, carboxy or aryl, such as phenyl, pyridyl or thienyl, groups;
Lys and Arg represent respectively peptide bonded lysyl and arginyl residues,

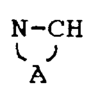

represents

* a bicyclic structure of the formula:

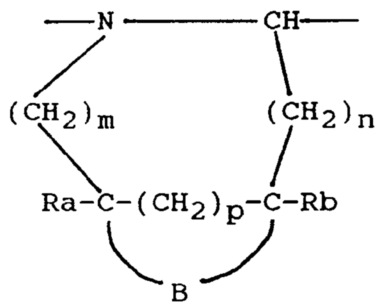

wherein
- m is 1 or 0,
- n and p represent 0, 1 or 2,
- Ra and Rb each represent a hydrogen atom or together can form a direct bond when p = 0,
- B represents:
  * an alkylene chain $(CH_2)q$ where q is 2, 3 or 4,
  * or an unsaturated structure $(-CH=CH-)_2$ when p = 0 and Ra and Rb together form a bond, with the proviso that the sum of m, n, p and q is an integer from 3 to 6, or
  * 1,2,3,4-tetrahydrobetacarboline,

their enantiomers, epimers and diastereoisomers,
and also their addition salts with a pharmaceutically acceptable acid or base,
characterised in that a compound of formula (II):

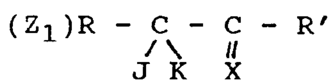 (II),

in which:
* R and X have the same meaning as in formula (I),
* R′ represents:
  - either a hydrogen atom or a hydroxy group when X represents an oxygen atom,
  - or a halogen atom and preferably a bromine atom, provided that in this case X represents two hydrogen atoms,
* J represents a hydrogen atom or a hydroxy group provided that K represents a hydrogen atom, or J represents a benzyloxycarbonylamino group provided that both K and R′ represent hydrogen atoms, or J and K together represent an oxygen atom provided that X represents two hydrogen atoms,
* $(Z_1)$ represents a group protecting any amino substituents of the radical R that may be present, especially the benzyloxycarbonyl group,

condensed with a compound of formula (III):

Lys(Z)-OtBu (III),

in which:
(Z) represents a group protecting the ω-amino substituent, especially the benzyloxycarbonyl group, and tBu represents a group protecting the carboxy substituent, especially the tert.-butyl radical,
in the presence:
- of a reducing agent, such as, for example, an alkali metal mixed hydride, such as, for example, an alkali metal borohydride or an alkali metal cyanoborohydride, when R′ represents a hydrogen atom,
- of a customary peptide coupling agent, such as dicyclohexylcarbodiimide in the presence of hydroxybenzotriazole, when R′ represents a hydroxy group, or
- of a basic agent, such as, for example, an alkali metal salt, such as, for example, an alkali metal carbonate, such as sodium carbonate, when R′ represents a halogen atom,

to obtain a compound of formula (IV):

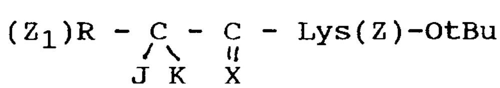 (IV)

in which R and X have the same meaning as in formula (I), J, K and $(Z_1)$ have the same meaning as in formula

(II), and (Z) and tBu have the same meaning as in formula (III), which compound (IV) is deprotected in an acidic medium at the carboxylic acid function of the lysine to form a compound of formula (V):

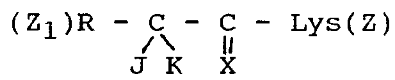 (V)

in which R and X have the same meaning as in formula (I), J, K and $(Z_1)$ have the same meaning as in formula (II), and (Z) has the same meaning as in formula (III),
which compound (V) is then condensed with a compound of formula (VI):

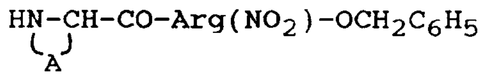 (VI),

in which A, together with the carbon and nitrogen atoms to which it is bonded, has the same meaning as in formula (I),
which is itself obtained by condensation of a compound of formula (VII):

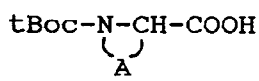 (VII),

in which A, together with the carbon and nitrogen atoms to which it is bonded, has the same meaning as in formula (I) and tBoc represents the tert.-butoxycarbonyl radical,
with benzyl $N_\omega$-nitroarginate ($H-Arg(NO_2)-OCH_2C_6H_5$) to obtain a compound of formula (VIII):

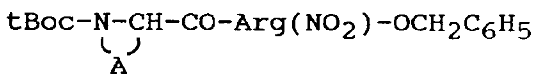 (VIII),

in which A, together with the carbon and nitrogen atoms to which it is bonded, has the same meaning as in formula (I),
which compound (VIII) is then deprotected by means of trifluoroacetic acid to form a compound of formula (VI), it being possible by condensation of the compound of formula (V) with the compound of formula (VI) to obtain a compound of formula (IX):

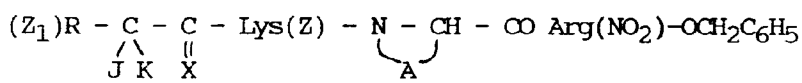 (IX),

in which A, together with the carbon and nitrogen atoms to which it is bonded, R and X have the same meaning as in formula (I), J, K and $(Z_1)$ have the same meaning as in formula (II), and (Z) has the same meaning as in formula (III),
which compound (IX), when J and K together represent an oxygen atom, is subjected to the action of an alkali metal mixed hydride, such as sodium borohydride, it being possible, if desired, to separate the resulting compound into its isomers by a conventional separation method, such as chromatography on a silica column,
to obtain a compound of the general formula (IXa), which is a special case of the compounds of formula (IX), in which A, together with the carbon and nitrogen atoms to which it is bonded, R, X, $(Z_1)$ and (Z) have the same meaning as in formula (IX), J represents a hydroxy group and K represents a hydrogen atom,
which compound of formula (IX) or (IXa) is deprotected by hydrogenolysis in an acidic polar solvent in the presence of a hydrogenation catalyst,
to give a compound of formula (I) which may, if desired:

- either be converted into a salt by means of a pharmaceutically acceptable acid or base,
- or be separated into its isomers and then, if necessary, converted into a salt by means of a pharmaceutically acceptable acid or base.

2. Process according to claim 1 for the preparation of compounds in which the cyclic structure

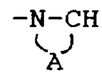

represents indoline, isoindoline, tetrahydroquinoline, tetrahydroisoquinoline, perhydroindole, perhydroisoindole, perhydroquinoline, perhydroisoquinoline, perhydrocyclopenta[b]pyrrole, 2-azabicyclo[2.2.2]octane, 2-azabicyclo[2.2.1]heptane, or 1,2,3,4-tetrahydrobetacarboline,
their isomers, epimers and diastereoisomers, and also their addition salts with a pharmaceutically acceptable acid or base.

3. Process according to either claim 1 or claim 2 for the preparation of compounds in which the cyclic structure

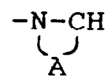

represents perhydroindole or azabicyclo[2.2.2]octane, their enantiomers, epimers and diastereoisomers, and also their addition salts with a pharmaceutically acceptable acid or base.

4. Process according to one of claims 1 to 3 for the preparation of AHPA-(S)Lys-(S)PHI-(S)Arg-OH, its isomers and its addition salts with a pharmaceutically acceptable acid or base.

5. Process according to one of claims 1 to 3 for the preparation of (2S,3R)AHPA-(S)Lys-(S)PHI-(S)Arg-OH and its addition salts with a pharmaceutically acceptable acid or base.

6. Process according to one of claims 1 to 3 for the preparation of AHPA-(S)Lys-(S)ABO-(S)Arg-OH, its isomers and its addition salts with a pharmaceutically acceptable acid or base.

7. Process according to one of claims 1 to 3 for the preparation of N-[2-hydroxy-3(R)-aminobutyryl]-(S)Lys-(S)ABO-(S)Arg-OH, its isomers and its addition salts with a pharmaceutically acceptable acid or base.

8. Process according to one of claims 1 to 3 for the preparation of (3-phenyl-2(S)-aminopropyl)-(S)Lys-(S)ABO-(S)Arg-OH, its addition salts with a pharmaceutically acceptable acid or base.

9. Process according to one of claims 1 to 3 for the preparation of (2-hydroxy-3(S)-amino-4-phenylbutyl)(S)Lys-(S)ABO-(S)Arg-OH, its isomers and its addition salts with a pharmaceutically acceptable acid or base.

10. Process according to one of claims 1 to 3 for the preparation of (2-hydroxy-3(R)-amino-4-phenylbutyl)(S)Lys-(S)ABO-(S)Arg-OH, its isomers and its addition salts with a pharmaceutically acceptable acid or base.

11. Process for the preparation of a pharmaceutical composition containing as active ingredient at least one compound according to one of claims 1 to 10 in combination with one or more pharmaceutically acceptable inert, non-toxic excipients or vehicles.

**CLAIMS for the Contracting State: ES**

1. Process for the preparation of compounds of the general formula (I):

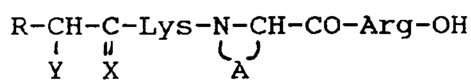

(I)

in which:
X represents:
- either an oxygen atom,
- or two hydrogen atoms,
Y represents:
- either a hydrogen atom,
- or a hydroxy group,
or
Y represents an amino group provided that X represents two hydrogen atoms;
R represents a hydrogen atom, a straight-chained or branched-chained alkyl group having from 1 to 6 carbon atoms that is optionally substituted by one or more hydroxy, amino, mercapto, methylthio, carboxy or aryl, such as phenyl, pyridyl or thienyl, groups;

Lys and Arg represent respectively peptide bonded lysyl and arginyl residues,

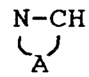

represents

* a bicyclic structure of the formula:

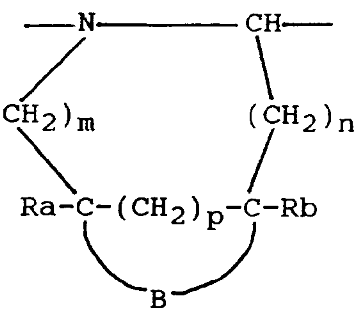

wherein

- m is 1 or 0,
- n and p represent 0, 1 or 2,
- Ra and Rb each represent a hydrogen atom or together can form a direct bond when p = 0,
- B represents:
    * an alkylene chain $(CH_2)q$ where q is 2, 3 or 4,
    * or an unsaturated structure $(-CH=CH-)_2$ when p = 0 and Ra and Rb together form a bond, with the proviso that the sum of m, n, p and q is an integer from 3 to 6, or
    * 1,2,3,4-tetrahydrobetacarboline,

their enantiomers, epimers and diastereoisomers,
and also their addition salts with a pharmaceutically acceptable acid or base,
characterised in that a compound of formula (II):

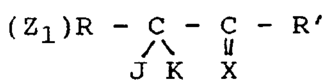

(II),

in which:

* R and X have the same meaning as in formula (I),
* R′ represents:
    - either a hydrogen atom or a hydroxy group when X represents an oxygen atom,
    - or a halogen atom and preferably a bromine atom, provided that in this case X represents two hydrogen atoms,
* J represents a hydrogen atom or a hydroxy group provided that K represents a hydrogen atom, or J represents a benzyloxycarbonylamino group provided that both K and R′ represent hydrogen atoms, or J and K together represent an oxygen atom provided that X represents two hydrogen atoms,
* $(Z_1)$ represents a group protecting any amino substituents of the radical R that may be present, especially the benzyloxycarbonyl group,

is condensed with a compound of formula (III):

Lys(Z)-OtBu (III),

in which:
(Z) represents a group protecting the ω-amino substituent, especially the benzyloxycarbonyl group, and tBu represents a group protecting the carboxy substituent, especially the tert.-butyl radical,
in the presence:

- of a reducing agent, such as, for example, an alkali metal mixed hydride, such as, for example, an alkali metal borohydride or an alkali metal cyanoborohydride, when R′ represents a hydrogen atom,
- of a customary peptide coupling agent, such as dicyclohexylcarbodiimide in the presence of hydroxyben-

zotriazole, when R′ represents a hydroxy group, or

– of a basic agent, such as, for example, an alkali metal salt, such as, for example, an alkali metal carbonate, such as sodium carbonate, when R′ represents a halogen atom,

to obtain a compound of formula (IV):

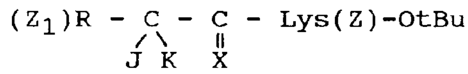

(IV)

in which R and X have the same meaning as in formula (I), J, K and ($Z_1$) have the same meaning as in formula (II), and (Z) and tBu have the same meaning as in formula (III),
which compound (IV) is deprotected in an acidic medium at the carboxylic acid function of the lysine to form a compound of formula (V):

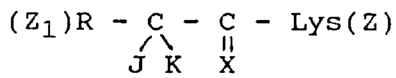

(V)

in which R and X have the same meaning as in formula (I), J, K and ($Z_1$) have the same meaning as in formula (II), and (Z) has the same meaning as in formula (III),
which compound (V) is then condensed with a compound of formula (VI):

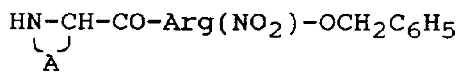

(VI),

in which A, together with the carbon and nitrogen atoms to which it is bonded, has the same meaning as in formula (I),
which is itself obtained by condensation of a compound of formula (VII):

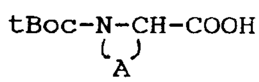

(VII),

in which A, together with the carbon and nitrogen atoms to which it is bonded, has the same meaning as in formula (I) and tBoc represents the tert.-butoxycarbonyl radical,
with benzyl $N_\omega$-nitroarginate (H-Arg($NO_2$)-$OCH_2C_6H_5$) to obtain a compound of formula (VIII):

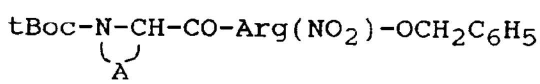

(VIII),

in which A, together with the carbon and nitrogen atoms to which it is bonded, has the same meaning as in formula (I),
which compound (VIII) is then deprotected by means of trifluoroacetic acid to form a compound of formula (VI), it being possible by condensation of the compound of formula (V) with the compound of formula (VI) to obtain a compound of formula (IX):

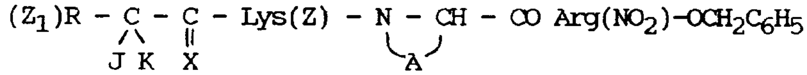

(IX),

in which A, together with the carbon and nitrogen atoms to which it is bonded, R and X have the same meaning as in formula (I), J, K and ($Z_1$) have the same meaning as in formula (II), and (Z) has the same meaning as in formula (III),
which compound (IX), when J and K together represent an oxygen atom, is subjected to the action of an alkali metal mixed hydride, such as sodium borohydride, it being possible, if desired, to separate the resulting compound into its isomers by a conventional separation method, such as chromatography on a silica column,

to obtain a compound of the general formula (IXa), which is a special case of the compounds of formula (IX), in which A, together with the carbon and nitrogen atoms to which it is bonded, R, X, ($Z_1$) and (Z) have the same meaning as in formula (IX), J represents a hydroxy group and K represents a hydrogen atom,
which compound of formula (IX) or (IXa) is deprotected by hydrogenolysis in an acidic polar solvent in the presence of a hydrogenation catalyst,
to give a compound of formula (I) which may, if desired:

- either be converted into a salt by means of a pharmaceutically acceptable acid or base,
- or be separated into its isomers and then, if necessary, converted into a salt by means of a pharmaceutically acceptable acid or base.

2. Process according to claim 1 for the preparation of compounds in which the cyclic structure

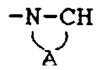

represents indoline, isoindoline, tetrahydroquinoline, tetrahydroisoquinoline, perhydroindole, perhydroisoindole, perhydroquinoline, perhydroisoquinoline, perhydrocyclopenta[b]pyrrole, 2-azabicyclo[2.2.2]octane, 2-azabicyclo[2.2.1]heptane, or 1,2,3,4-tetrahydrobetacarboline,
their isomers, epimers and diastereoisomers, and also their addition salts with a pharmaceutically acceptable acid or base.

3. Process according to either claim 1 or claim 2 for the preparation of compounds in which the cyclic structure

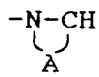

represents perhydroindole or azabicyclo[2.2.2]octane, their enantiomers, epimers and diastereoisomers, and also their addition salts with a pharmaceutically acceptable acid or base.

4. Process according to one of claims 1 to 3 for the preparation of AHPA-(S)Lys-(S)PHI-(S)Arg-OH, its isomers and its addition salts with a pharmaceutically acceptable acid or base.

5. Process according to one of claims 1 to 3 for the preparation of (2S,3R)AHPA-(S)Lys-(S)PHI-(S)Arg-OH and its addition salts with a pharmaceutically acceptable acid or base.

6. Process according to one of claims 1 to 3 for the preparation of AHPA-(S)Lys-(S)ABO-(S)Arg-OH, its isomers and its addition salts with a pharmaceutically acceptable acid or base.

7. Process according to one of claims 1 to 3 for the preparation of N-[2-hydroxy-3(R)-aminobutyryl]-(S)Lys-(S)ABO-(S)Arg-OH, its isomers and its addition salts with a pharmaceutically acceptable acid or base.

8. Process according to one of claims 1 to 3 for the preparation of (3-phenyl-2(S)-aminopropyl)-(S)Lys-(S)ABO-(S)Arg-OH, its addition salts with a pharmaceuticatly acceptable acid or base.

9. Process according to one of claims 1 to 3 for the preparation of (2-hydroxy-3(S)-amino-4-phenylbutyl)(S)Lys-(S)ABO-(S)Arg-OH, its isomers and its addition salts with a pharmaceutically acceptable acid or base.

10. Process according to one of claims 1 to 3 for the preparation of (2-hydroxy-3(R)-amino-4-phenylbutyl)(S)Lys-(S)ABO-(S)Arg-OH, its isomers and its addition salts with a pharmaceutically acceptable acid or base.

11. Process for the preparation of a pharmaceutical composition containing as active ingredient at least one compound according to one of claims 1 to 10 in combination with one or more pharmaceutically acceptable inert, non-toxic excipients or vehicles.

## Patentansprüche

### Patentansprüche für folgende Vertragsstaaten AT, BE, CH, DE, FR, GB, IT, LI, LU, NL, SE

1. Verbindungen der allgemeinen Formel

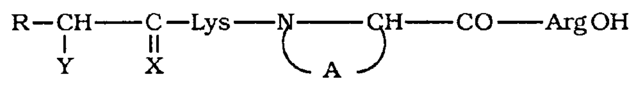

(I)

in der

X

- entweder ein Sauerstoffatom
- oder zwei Wasserstoffatome,

Y

- entweder ein Wasserstoffatom
- oder eine Hydroxylgruppe

oder

Y eine Aminogruppe dann, wenn X zwei Wasserstoffatome darstellt;

R ein Wasserstoffatom, eine geradkettige oder verzweigte Alkylgruppe mit 1 bis 6 Kohlenstoffatomen, die gegebenenfalls durch eine oder mehrere Hydroxygruppen, Aminogruppen, Mercaptogruppen, Methylthiogruppen, Carboxygruppen oder Arylgruppen, wie Phenylgruppen, Pyridylgruppen oder Thienylgruppen, substituiert ist;

Lys und Arg in Peptidbindungen vorliegende Lysyl- bzw. Arginyl-Reste;

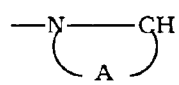

* eine bicyclische Struktur der Formel

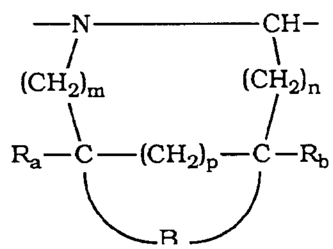

in der

- m 1 oder 0,
- n und p 0, 1 oder 2,
- Ra und Rb Wasserstoffatome oder gemeinsam eine direkte Bindung dann, wenn p = 0 ist,
- B :
  - * eine Alkylenkette der Formel $(CH_2)q$, worin q 2, 3 oder 4 darstellt,
  - * oder eine ungesättigte Struktur der Formel $(- CH = CH - )_2$, wenn p = 0 ist und Ra und Rb gemeinsam eine Bindung darstellen,

  mit der Maßgabe, daß die Summe m, n, p und q eine ganze Zahl zwischen 3 und 6 ist, oder

  * 1,2,3,4-Tetrahydro-betacarbolin

bedeuten

sowie deren Enantiomere, Epimere und Diastereoisomere,

sowie deren Additionssalze mit einer pharmazeutisch annehmbaren Säure oder Base.

2. Verbindungen nach Anspruch 1, worin die cyclische Gruppe der Formel

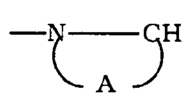

für einen Rest von Indolin, Isoindolin, Tetrahydrochinolin, Tetrahydroisochinolin, Perhydroindol, Perhydroisoindol, Perhydrochinolin, Perhydroisochinolin, Perhydrocyclopenta[b]pyrrol, 2-Aza-bicyclo[2.2.2]octan, 2-Aza-bicyclo[2.2.1]heptan oder 1,2,3,4-Tetrahydro-betacarbolin steht,

sowie deren Isomere, Epimere und Diastereoisomere sowie deren Additionssalze mit einer pharmazeutisch annehmbaren Säure oder Base.

3. Verbindungen nach einem der Ansprüche 1 oder 2, worin die cyclische Struktur der Formel

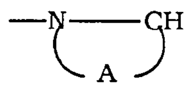

für den Perhydroindol- oder den Azabicyclo[2.2.2]octan-Rest steht, sowie deren Enantiomere, Epimere und Diastereoisomere sowie deren Additionssalze mit einer pharmazeutisch annehmbaren Säure oder Base.

4. AHPA - (S)Lys - (S)PHI - (S)Arg - OH, dessen Isomere und dessen Additionssalze mit einer pharmazeutisch annehmbaren Säure oder Base.

5. (2S, 3R)AHPA - (S)Lys - (S)PHI - (S)Arg - OH, dessen Isomere und dessen Additionssalze mit einer pharmazeutisch annehmbaren Säure oder Base.

6. AHPA - (S)Lys - (S)ABO - (S)Arg - OH, dessen Isomere und dessen Additionssalze mit einer pharmazeutisch annehmbaren Säure oder Base.

7. N-[2-Hydroxy-3(R)-amino-butyryl-(S)Lys - (S)ABO - (S)Arg - OH, dessen Isomere und dessen Additionssalze mit einer pharmazeutisch annehmbaren Säure oder Base.

8. (3-Phenyl-2(S)-amino-propyl) - (S)Lys - (S)ABO - (S)Arg - OH, dessen Isomere und dessen Additionssalze mit einer pharmazeutisch annehmbaren Säure oder Base.

9. (2-Hydroxy-3(S)-amino-4-phenyl-butyl) - (S)Lys - (S)ABO - (S)Arg - OH, dessen Isomere und dessen Additionssalze mit einer pharmazeutisch annehmbaren Säure oder Base.

10. (2 -Hydroxy-3(R)-amino-4-phenyl-butyl) - (S)Lys - (S)ABO - (S)Arg - OH, dessen Isomere und dessen Additionssalze mit einer pharmazeutisch annehmbaren Säure oder Base.

11. Verfahren zur Herstellung der Derivate der Formel I, **dadurch gekennzeichnet**, daß man ein Derivat der Formel (II)

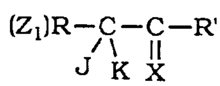

(II)

in der

- * R und X die bezüglich der Formel (I) angegebenen Bedeutungen besitzen,
- * R′
  - – entweder ein Wasserstoffatom oder eine Hydroxylgruppe dann, wenn X ein Sauerstoffatom darstellt,
  - – oder ein Halogenatom und vorzugsweise ein Bromatom dann, wenn X zwei Wasserstoffatome darstellt,
- * J ein Wasserstoffatom oder eine Hydroxylgruppe dann, wenn K ein Wasserstoffatom bedeutet, oder J eine Benzyloxycarbonylaminogruppe mit der doppelten Maßgabe, daß K und R′ jeweils ein Wasserstoffatom darstellen, oder J und K gemeinsam ein Sauerstoffatom mit der Maßgabe, daß X zwei Wasserstoffatome darstellt, und
- * ($Z_1$) eine Schutzgruppe für eventuelle Aminosubstituenten des Restes R und insbesondere die Benzyloxycarbonylgruppe bedeuten,

mit einem Derivat der Formel (III)

Lys(Z) - OtBu (III)

in der

(Z) eine Schutzgruppe des ω-Amino-Substituenten und insbesondere die Benzyloxycarbonylgruppe und tBu eine Schutzgruppe des Carboxylsubstituenten und insbesondere den tert.-Butylrest bedeuten, in Gegenwart:

- – eines Reduktionsmittels, wie beispielsweise eines gemischten Alkalimetallhydrids, wie beispielsweise eines Alkalimetallborhydrids oder eines Alkalimetallcyanoborhydrids dann, wenn R′ ein Wasserstoffatom darstellt,
- – eines üblichen Peptid-Kupplungsmittels, wie Dicyclohexylcarbodiimid in Gegenwart von Hydroxybenzotriazol dann, wenn R′ eine Hydroxylgruppe darstellt,
- – eines basischen Mittels, wie eines Alkalimetallsalzes, beispielsweise eines Alkalimetallcarbonats, wie Natriumcarbonat, dann, wenn R′ ein Halogenatom darstellt,

kondensiert zur Bildung eines Derivats der Formel (IV):

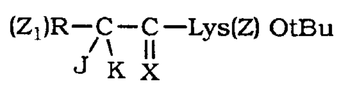

(IV)

in der R und X die bezüglich der allgemeinen Formel (I) angegebenen Bedeutungen besitzen, J, K und ($Z_1$)

die bezüglich der Formel (II) angegebenen Bedeutungen besitzen und (Z) und tBu die bezüglich der Formel (III) angegebenen Bedeutungen besitzen,
von welcher Verbindung man in saurem Medium die Schutzgruppe im Bereich der Carbonsäurefunktion des Lysins abspaltet unter Bildung eines Derivats der Formel (V):

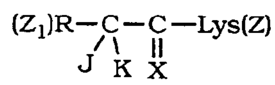

(V)

in der R und X die bezüglich der Formel (I) angegebenen Bedeutungen besitzen, J, K und ($Z_1$) die bezüglich der Formel (II) angegebenen Bedeutungen besitzen und (Z) die bezüglich der Formel (III) angegebenen Bedeutungen besitzt,
welches man anschließend mit einem Derivat der Formel (VI):

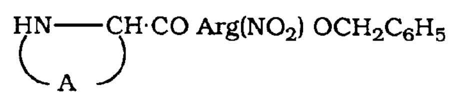

(VI)

in der A zusammen mit dem Kohlenstoff- und dem Stickstoffatom, an das es gebunden ist, die gleiche Bedeutung besitzt wie sie bezüglich der Formel (I) angegeben ist,
welches man seinerseits durch Kondensation eines Derivats der Formel (VII):

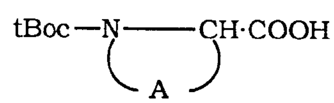

(VII)

in der A zusammen mit dem Kohlenstoff- und dem Stickstoffatom, an die es gebunden ist, die gleiche Bedeutung wie die bezüglich der Formel (I) angegebene besitzt, und tBoc für den tert.-Butoxycarbonylrest steht, mit Benzyl-Nω-nitroarginat (H-Arg($NO_2$)$OCH_2C_6H_5$) unter Bildung eines Derivats der Formel (VIII):

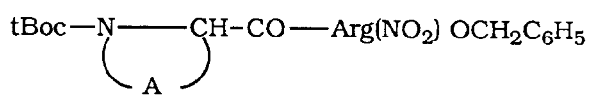

(VIII)

in dem A zusammen mit dem Kohlenstoff- und dem Stickstoffatom, an die es gebunden ist, die gleiche Bedeutung besitzt wie sie für die Formel (I) angegeben ist,
von welchem man anschließend mit Trifluoressigsäure die Schutzgruppe abspaltet unter Bildung eines Derivats der Formel (VI), erhalten hat, wobei die Kondensation des Derivats der Formel (V) mit dem Derivat der Formel (VI) zu einem Derivat der Formel (IX):

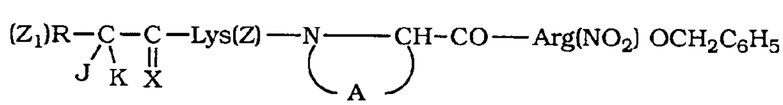

(IX)

führt, in der A zusammen mit dem Kohlenstoff- und dem Stickstoffatom, an die es gebunden ist, sowie R und X die bezüglich der Formel (I) angegebenen Bedeutungen besitzen, J, K und ($Z_1$) die bezüglich der Formel (II) angegebenen Bedeutungen besitzen und (Z) die bezüglich der Formel (III) angegebenen Bedeutungen besitzt, welches dann, wenn J und K gemeinsam ein Sauerstoffatom darstellen, der Einwirkung eines gemischten Alkalimetallhydrids, wie Natriumborhydrid, unterworfen wird, wobei die in dieser Weise erhaltene Verbindung gewünschtenfalls mit Hilfe klassischer Trennverfahren, wie der Säulenchromatographie über Siliciumdioxid in die Isomeren aufgetrennt werden kann,
unter Bildung einer Verbindung der allgemeinen Formel (IXa), welche einen besonderen Fall der Derivate der Formel (IX) darstellt, in der A zusammen mit dem Kohlenstoff- und dem Stickstoffatom, an die es gebunden

ist, R, X, ($Z_1$) und (Z) die bezüglich der Formel (IX) angegebenen Bedeutungen besitzen, J eine Hydroxylgruppe und K ein Wasserstoffatom bedeuten,
von welchem Derivat der Formel (IX) oder (IXa) man durch Hydrogenolyse in einem sauren polaren Lösungsmittel in Gegenwart eines Hydrierkatalysators die Schutzgruppe abspaltet,
zur Bildung eines Derivats der Formel (I), welches man gewünschtenfalls:

- entweder mit einer pharmazeutisch annehmbaren Säure oder Base in ein Salz überführen
- oder in die Isomeren auftrennen kann, die man gewünschtenfalls mit einer pharmazeutisch annehmbaren Säure oder Base in Salze überführen kann.

12. Verbindungen der allgemeinen Formeln (IX) und (IXa) nach Anspruch 11 zur Verwendung bei der Herstellung von Verbindungen nach Anspruch 1.

13. Pharmazeutische Zubereitung enthaltend als Wirkstoff mindestens eine Verbindung nach einem der Ansprüche 1 bis 10 in Kombination mit einem oder mehreren inerten, nichttoxischen, pharmazeutisch annehmbaren Bindemitteln oder Trägermaterialien.

14. Pharmazeutische Zubereitung nach Anspruch 13 zur Behandlung von Erkrankungen, die mit einer Störung des Immunschutzes des Organismus verbunden sind und insbesondere zur Behandlung von Krebs, durch Viren, Bakterien oder Pilze verursachten Erkrankungen, Autoimmunerkrankungen, wie Lupus erythematodes oder rheumatische Arthritis.

**Patentansprüche für folgenden Vertragsstaat: GR**

1. Verfahren zur Herstellung von Verbindungen der allgemeinen Formel (I):

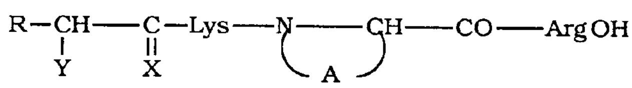

(I)

in der
X

- entweder ein Sauerstoffatom
- oder zwei Wasserstoffatome,

Y

- entweder ein Wasserstoffatom
- oder eine Hydroxylgruppe

oder Y eine Aminogruppe dann, wenn X zwei Wasserstoffatome darstellt;
R ein Wasserstoffatom, eine geradkettige oder verzweigte Alkylgruppe mit 1 bis 6 Kohlenstoffatomen, die gegebenenfalls durch eine oder mehrere Hydroxygruppen, Aminogruppen, Mercaptogruppen, Methylthiogruppen, Carboxygruppen oder Arylgruppen, wie Phenylgruppen, Pyridylgruppen oder Thienylgruppen, substituiert ist;
Lys und Arg in Peptidbindungen vorliegende Lysyl- bzw. Arginyl-Reste;

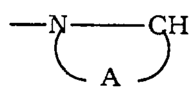

* eine bicyclische Struktur der Formel

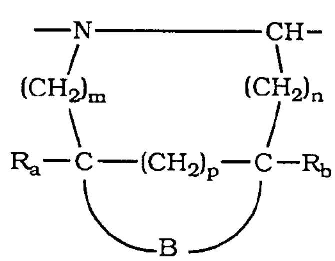

in der

- m 1 oder 0,
- n und p 0, 1 oder 2,
- Ra und Rb Wasserstoffatome oder gemeinsam eine direkte Bindung dann, wenn p = 0 ist,

– B:

* eine Alkylenkette der Formel $(CH_2)q$, worin q 2, 3 oder 4 darstellt,
* oder eine ungesättigte Struktur der Formel $(- CH = CH -)_2$, wenn p = 0 ist und Ra und Rb gemeinsam eine Bindung darstellen, mit der Maßgabe, daß die Summe m, n, p und q eine ganze Zahl zwischen 3 und 6 ist, oder * 1,2,3,4-Tetrahydro-betacarbolin

bedeuten,
sowie von deren Enantiomeren, Epimeren und Diastereoisomeren, sowie von deren Additionssalzen mit einer pharmazeutisch annehmbaren Säure oder Base,
dadurch gekennzeichnet, daß man ein Derivat der Formel (II)

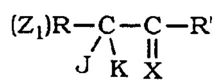

(II)

in der

* R und X die bezüglich der Formel (I) angegebenen Bedeutungen besitzen,
* R′
  – entweder ein Wasserstoffatom oder eine Hydroxylgruppe dann, wenn X ein Sauerstoffatom darstellt,
  – oder ein Halogenatom und vorzugsweise ein Bromatom dann, wenn X zwei Wasserstcffatome darstellt,
* J ein Wasserstoffatom oder eine Hydroxylgruppe dann, wenn K ein Wasserstoffatom bedeutet, oder J eine Benzyloxycarbonylaminogruppe mit der doppelten Maßgabe, daß K und R′ jeweils ein Wasserstoffatom darstellen, oder
  J und K gemeinsam ein Sauerstoffatom mit der Maßgabe, daß X zwei Wasserstoffatome darstellt, und
* $(Z_1)$ eine Schutzgruppe für eventuelle Aminosubstituenten des Restes R und insbesondere die Benzyloxycarbonylgruppe bedeuten,

mit einem Derivat der Formel (III)

Lys(Z) - OtBu (III)

in der
(Z) eine Schutzgruppe des ω-Amino-Substituenten und insbesondere die Benzyloxycarbonylgruppe und tBu eine Schutzgruppe des Carboxylsubstituenten und insbesondere den tert.-Butylrest bedeuten,
in Gegenwart:

– eines Reduktionsmittels, wie beispielsweise eines gemischten Alkalimetallhydrids, wie beispielsweise eines Alkalimetallborhydrids oder eines Alkalimetallcyanoborhydrids dann, wenn R′ ein Wasserstoffatom darstellt,
– eines üblichen Peptid-Kupplungsmittels, wie Dicyclohexylcarbodiimid in Gegenwart von Hydroxybenzotriazol dann, wenn R′ eine Hydroxylgruppe darstellt,
– eines basischen Mittels, wie eines Alkalimetallsalzes, beispielsweise eines Alkalimetallcarbonats, wie Natriumcarbonat, dann, wenn R'ein Halogenatom darstellt,

kondensiert zur Bildung eines Derivats der Formel (IV):

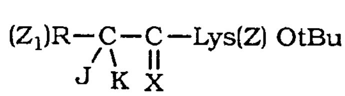

(IV)

in der R und X die bezüglich der allgemeinen Formel (I) angegebenen Bedeutungen besitzen, J, K und $(Z_1)$ die bezüglich der Formel (II) angegebenen Bedeutungen besitzen und (Z) und tBu die bezüglich der Formel (III) angegebenen Bedeutungen besitzen,
von welcher Verbindung man in saurem Medium die Schutzgruppe im Bereich der Carbonsäurefunktion des Lysins abspaltet unter Bildung eines Derivats der Formel (V):

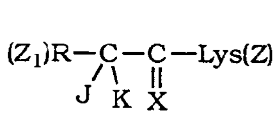

(V)

in der R und X die bezüglich der Formel (I) angegebenen Bedeutungen besitzen, J, K und $(Z_1)$ die bezüglich der Formel (II) angegebenen Bedeutungen besitzen und (Z) die bezüglich der Formel (III) angegebenen Bedeutungen besitzt,
welches man anschließend mit einem Derivat der Formel (VI):

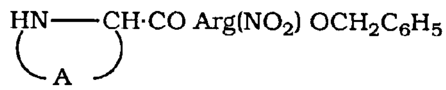 (VI)

in der A zusammen mit dem Kohlenstoff- und dem Stickstoffatom, an das es gebunden ist, die gleiche Bedeutung besitzt wie sie bezüglich der Formel (I) angegeben ist,
welches man seinerseits durch Kondensation eines Derivats der Formel (VII):

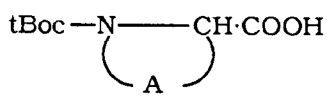 (VII)

in der A zusammen mit dem Kohlenstoff- und dem Stickstoffatom, an die es gebunden ist, die gleiche Bedeutung wie die bezüglich der Formel (I) angegebene besitzt, und tBoc für den tert.-Butoxycarbonylrest steht,
mit Benzyl-Nω-nitroarginat (H-Arg($NO_2$)$OCH_2C_6H_5$) unter Bildung eines Derivats der Formel (VIII):

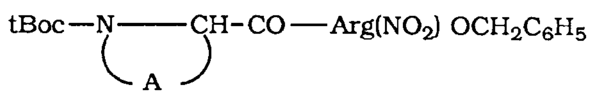 (VIII)

in dem A zusammen mit dem Kohlenstoff- und dem Stickstoffatom, an die es gebunden ist, die gleiche Bedeutung besitzt wie sie für die Formel (I) angegeben ist,
von welchem man anschließend mit Trifluoressigsäure die Schutzgruppe abspaltet unter Bildung eines Derivats der Formel (VI), erhalten hat wobei die Kondensation des Derivats der Formel (V) mit dem Derivat der Formel (VI) zu einem Derivat der Formel (IX):

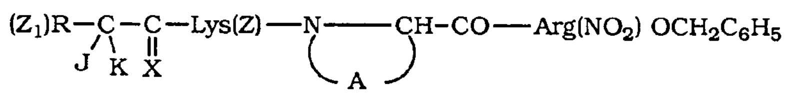 (IX)

führt, in der A zusammen mit dem Kohlenstoff- und dem Stickstoffatom, an die es gebunden ist, sowie R und X die bezüglich der Formel (I) angegebenen Bedeutungen besitzen, J, K und ($Z_1$) die bezüglich der Formel (II) angegebenen Bedeutungen besitzen und (Z) die bezüglich der Formel (III) angegebenen Bedeutungen besitzt,
welches dann, wenn J und K gemeinsam ein Sauerstoffatom darstellen, der Einwirkung eines gemischten Alkalimetallhydrids, wie Natriumborhydrid, unterworfen wird, wobei die in dieser Weise erhaltene Verbindung gewünschtenfalls mit Hilfe klassischer Trennverfahren, wie der Säulenchromatographie über Siliciumdioxid in die Isomeren aufgetrennt werden kann,
unter Bildung einer Verbindung der allgemeinen Formel (IXa), welche einen besonderen Fall der Derivate der Formel (IX) darstellt, in der A zusammen mit dem Kohlenstoff- und dem Stickstoffatom, an die es gebunden ist, R, X, ($Z_1$) und (Z) die bezüglich der Formel (IX) angegebenen Bedeutungen besitzen, J eine Hydroxylgruppe und K ein Wasserstoffatom bedeuten,
von welchem Derivat der Formel (IX) oder (IXa) man durch Hydrogenolyse in einem sauren polaren Lösungsmittel in Gegenwart eines Hydrierkatalysators die Schutzgruppe abspaltet,
zur Bildung eines Derivats der Formel (I), welches man gewünschtenfalls:

- entweder mit einer pharmazeutisch annehmbaren Säure oder Base in ein Salz überführen
- oder in die Isomeren auftrennen kann, die man gewünschtenfalls mit einer pharmazeutisch annehmbaren Säure oder Base in Salze überführen kann.

2. Verfahren nach Anspruch 1 zur Herstellung von Verbindungen, in denen die cyclische Struktur der Formel

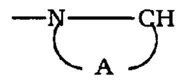

für einen Rest von Indolin, Isoindolin, Tetrahydrochinolin, Tetrahydroisochinolin, Perhydroindol, Perhydroisoindol, Perhydrochinolin, Perhydroisochinolin, Perhydrocyclopenta[b]pyrrol, 2-Aza-bicyclo[2.2.2]octan, 2-Azabicyclo[2.2.1]heptan oder 1,2,3,4-Tetrahydro-betacarbolin steht, sowie deren Isomeren, Epimeren und Diastereoisomeren sowie deren Additionssalzen mit einer pharmazeutisch annehmbaren Säure oder Base.

3. Verfahren nach einem der Ansprüche 1 oder 2 zur Herstellung von Verbindungen, in denen die cyclische Struktur der Formel

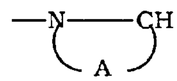

für den Perhydroindol- oder den Azabicyclo[2.2.2] octan-Rest steht, sowie deren Enantiomeren, Epimeren und Diastereoisomeren sowie deren Additionssalze mit einer pharmazeutisch annehmbaren Säure oder Base.

4. Verfahren nach einem der Ansprüche 1 bis 3 zur Herstellung von AHPA - (S)Lys - (S)PHI - (S)Arg - OH, dessen Isomeren und dessen Additionssalzen mit einer pharmazeutisch annehmbaren Säure oder Base.

5. Verfahren nach einem der Ansprüche 1 bis 3 zur Herstellung von (2S, 3R)AHPA - (S)Lys - (S)PHI - (S)Arg - OH, dessen Isomeren und dessen Additionssalzen mit einer pharmazeutisch annehmbaren Säure oder Base.

6. Verfahren nach einem der Ansprüche 1 bis 3 zur Herstellung von AHPA - (S)Lys - (S)ABO - (S)Arg - OH, dessen Isomeren und dessen Additionssalzen mit einer pharmazeutisch annehmbaren Säure oder Base.

7. Verfahren nach einem der Ansprüche 1 bis 3 zur Herstellung von N-[2-Hydroxy-3(R)-amino-butyryl]-(S)Lys - (S)ABO - (S)Arg - OH, dessen Isomeren und dessen Additionssalzen mit einer pharmazeutisch annehmbaren Säure oder Base.

8. Verfahren nach einem der Ansprüche 1 bis 3 zur Herstellung von (3-Phenyl-2(S)-amino-propyl) - (S)Lys - (S)ABO - (S)Arg - OH, dessen Isomeren und dessen Additionssalzen mit einer pharmazeutisch annehmbaren Säure oder Base.

9. Verfahren nach einem der Ansprüche 1 bis 3 zur Herstellung von (2-Hydroxy-3(S)-amino-4-phenyl-butyl) - (S)Lys - (S)ABO - (S)Arg - OH, dessen Isomeren und dessen Additionssalzen mit einer pharmazeutisch annehmbaren Säure oder Base.

10. Verfahren nach einem der Ansprüche 1 bis 3 zur Herstellung von (2-Hydroxy-3(R)-amino-4-phenyl-butyl) - (S)Lys - (S)ABO - (S)Arg - OH, dessen Isomeren und dessen Additionssalzen mit einer pharmazeutisch annehmbaren Säure oder Base.

11. Verfahren zur Herstellung einer pharmazeutischen Zubereitung enthaltend als Wirkstoffmindestens eine Verbindung nach einem der Ansprüche 1 bis 10 in Kombination mit einem oder mehreren inerten, nichttoxischen, pharmazeutisch annehmbaren Bindemitteln oder Trägermaterialien.

**Patentansprüche für folgenden Vertragsstaat: ES**

1. Verfahren zur Herstellung von Verbindungen der allgemeinen Formel (I):

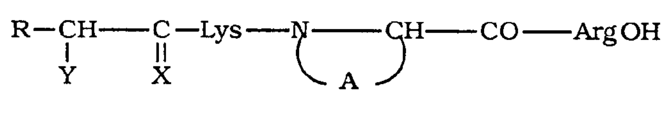

(I)

in der

X
- entweder ein Sauerstoffatom
- oder zwei Wasserstoffatome,

Y
- entweder ein Wasserstoffatom
- oder eine Hydroxylgruppe

oder

Y eine Aminogruppe dann, wenn X zwei Wasserstoffatome darstellt;

R ein Wasserstoffatom, eine geradkettige oder verzweigte Alkylgruppe mit 1 bis 6 Kohlenstoffatomen, die gegebenenfalls durch eine oder mehrere Hydroxygruppen, Aminogruppen, Mercaptogruppen, Methylthiogruppen,

Carboxygruppen oder Arylgruppen, wie Phenylgruppen, Pyridylgruppen oder Thienylgruppen, substituiert ist; Lys und Arg in Peptidbindungen vorliegende Lysyl- bzw. Arginyl-Reste;

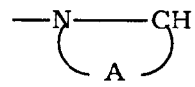

* eine bicyclische Struktur der Formel

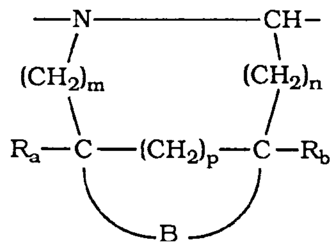

in der

- m 1 oder 0,
- n und p 0, 1 oder 2,
- Ra und Rb Wasserstoffatome oder gemeinsam eine direkte Bindung dann, wenn p = 0 ist,
- B
  - * eine Alkylenkette der Formel $(CH_2)q$, worin q 2, 3 oder 4 darstellt,
  - * oder eine ungesättigte Struktur der Formel $(- CH = CH - )_2$, wenn p = 0 ist und Ra und Rb gemeinsam eine Bindung darstellen,

  mit der Maßgabe, daß die Summe m, n, p und q eine ganze Zahl zwischen 3 und 6 ist, oder

  * 1,2,3,4-Tetrahydro-betacarbolin

bedeuten

sowie von deren Enantiomeren, Epimeren und Diastereoisomeren, sowie von deren Additionssalzen mit einer pharmazeutisch annehmbaren Säure oder Base,

**dadurch gekennzeichnet**, daß man ein Derivat der Formel (II)

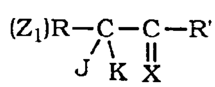 (II)

in der

* R und X die bezüglich der Formel (I) angegebenen Bedeutungen besitzen,

* R′

- entweder ein Wasserstoffatom oder eine Hydroxylgruppe dann, wenn X ein Sauerstoffatom darstellt,
- oder ein Halogenatom und vorzugsweise ein Bromatom dann, wenn X zwei Wasserstoffatome darstellt,

* J ein Wasserstoffatom oder eine Hydroxylgruppe dann, wenn K ein Wassserstoffatom bedeutet, oder J eine Benzyloxycarbonylaminogruppe mit der doppelten Maßgabe, daß K und R′ jeweils ein Wasserstoffatom darstellen, oder

J und K gemeinsam ein Sauerstoffatom mit der Maßgabe, daß X zwei Wasserstoffatome darstellt, und

* $(Z_1)$ eine Schutzgruppe für eventuelle Aminosubstituenten des Restes R und insbesondere die Benzyloxycarbonylgruppe bedeuten,

mit einem Derivat der Formel (III)

Lys(Z) - OtBu (III)

in der

(Z) eine Schutzgruppe des ω-Amino-Substituenten und insbesondere die Benzyloxycarbonylgruppe und tBu eine Schutzgruppe des Carboxylsubstituenten und insbesondere den tert.-Butylrest bedeuten,

in Gegenwart:

- eines Reduktionsmittels, wie beispielsweise eines gemischten Alkalimetallhydrids, wie beispielsweise eines Alkalimetallborhydrids oder eines Alkalimetallcyanoborhydrids dann, wenn R′ ein Sauerstoffatom darstellt,

– eines üblichen Peptid-Kupplungsmittels, wie Dicyclohexylcarbodiimid in Gegenwart von Hydroxybenzotriazol dann, wenn R′ eine Hydroxylgruppe darstellt,

– eines basischen Mittels, wie eines Alkalimetallsalzes, beispielsweise eines Alkalimetallcarbonats, wie Natriumcarbonat, dann, wenn R′ ein Halogenatom darstellt,

kondensiert zur Bildung eines Derivats der Formel (IV):

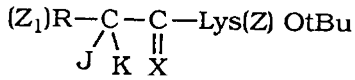 (IV)

in der R und X die bezüglich der allgemeinen Formel (I) angegebenen Bedeutungen besitzen, J, K und ($Z_1$) die bezüglich der Formel (II) angegebenen Bedeutungen besitzen und (Z) und tBu die bezüglich der Formel (III) angegebenen Bedeutungen besitzen,
von welcher Verbindung man in saurem Medium die Schutzgruppe im Bereich der Carbonsäurefunktion des Lysins abspaltet unter Bildung eines Derivats der Formel (V):

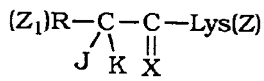 (V)

in der R und X die bezüglich der Formel (I) angegebenen Bedeutungen besitzen, J, K und ($Z_1$) die bezüglich der Formel (II) angegebenen Bedeutungen besitzen und (Z) die bezüglich der Formel (III) angegebenen Bedeutungen besitzt,
welches man anschließend mit einem Derivat der Formel (VI):

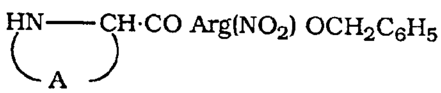 (VI)

in der A zusammen mit dem Kohlenstoff- und dem Stickstoffatom, an das es gebunden ist, die gleiche Bedeutung besitzt wie sie bezüglich der Formel (I) angegeben ist,
welches man seinerseits durch Kondensation eines Derivats der Formel (VII):

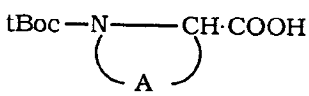 (VII)

in der A zusammen mit dem Kohlenstoff- und dem Stickstoffatom, an die es gebunden ist, die gleiche Bedeutung wie die bezüglich der Formel (I) angegebene besitzt, und tBoc für den tert.-Butoxycarbonylrest steht,
mit Benzyl-$N_\omega$-nitroarginat (H-Arg($NO_2$)$OCH_2C_6H_5$) unter Bildung eines Derivats der Formel (VIII):

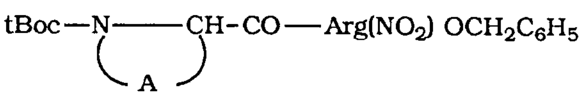 (VIII)

in dem A zusammen mit dem Kohlenstoff- und dem Stickstoffatom, an die es gebunden ist, die gleiche Bedeutung besitzt wie sie für die Formel (I) angegeben ist,
von welchem man anschließend mit Trifluoressigsäure die Schutzgruppe abspaltet unter Bildung eines Derivats der Formel (VI), erhalten hat,
wobei die Kondensation des Derivats der Formel (V) mit dem Derivat der Formel (VI) zu einem Derivat der Formel (IX):

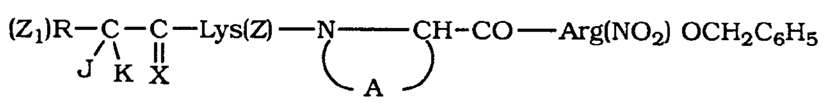 (IX)

führt, in der A zusammen mit dem Kohlenstoff- und dem Stickstoffatom, an die es gebunden ist, sowie R und X die bezüglich der Formel (I) angegebenen Bedeutungen besitzen, J, K und $(Z_1)$ die bezüglich der Formel (II) angegebenen Bedeutungen besitzen und (Z) die bezüglich der Formel (III) angegebenen Bedeutungen besitzt, welches dann, wenn J und K gemeinsam ein Sauerstoffatom darstellen, der Einwirkung eines gemischten Alkalimetallhydrids, wie Natriumborhydrid, unterworfen wird, wobei die in dieser Weise erhaltene Verbindung gewünschtenfalls mit Hilfe klassischer Trennverfahren, wie der Säulenchromatographie über Siliciumdioxid in die Isomeren aufgetrennt werden kann,
unter Bildung einer Verbindung der allgemeinen Formel (IXa), welche einen besonderen Fall der Derivate der Formel (IX) darstellt, in der A zusammen mit dem Kohlenstoff- und dem Stickstoffatom, an die es gebunden ist, R, X, $(Z_1)$ und (Z) die bezüglich der Formel (IX) angegebenen Bedeutungen besitzen, J eine Hydroxylgruppe und K ein Wasserstoffatom bedeuten,
von welchem Derivat der Formel (IX) oder (IXa) man durch Hydrogenolyse in einem sauren polaren Lösungsmittel in Gegenwart eines Hydrierkatalysators die Schutzgruppe abspaltet, zur Bildung eines Derivats der Formel (I), welches man gewünschtenfalls:

– entweder mit einer pharmazeutisch annehmbaren Säure oder Base in ein Salz überführen
– oder in die Isomeren auftrennen kann, die man gewünschtenfalls mit einer pharmazeutisch annehmbaren Säure oder Base in Salze überführen kann.

2. Verfahren nach Anspruch 1 zur Herstellung von Verbindungen, in denen die cyclische Struktur der Formel

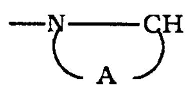

für einen Rest von Indolin, Isoindolin, Tetrahydrochinolin, Tetrahydroisochinolin, Perhydroindol, Perhydroisoindol, Perhydrochinolin, Perhydroisochinolin, Perhydrocyclopenta[b]pyrrol, 2-Aza-bicyclo[2.2.2]octan, 2-Aza-bicyclo[2.2.1]heptan oder 1,2,3,4-Tetrahydro-betacarbolin steht, sowie deren Isomeren, Epimeren und Diastereoisomeren sowie deren Additionssalzen mit einer pharmazeutisch annehmbaren Säure oder Base.

3. Verfahren nach einem der Ansprüche 1 oder 2 zur Herstellung von Verbindungen, in denen die cyclische Struktur der Formel

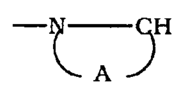

für den Perhydroindol- oder den Azabicyclo[2.2.2]octan-Rest steht, sowie deren Enantiomeren, Epimeren und Diastereoisomeren sowie deren Additionssalzen mit einer pharmazeutisch annehmbaren Säure oder Base.

4. Verfahren nach einem der Ansprüche 1 bis 3 zur Herstellung von AHPA - (S)Lys - (S)PHI - (S)Arg - OH, dessen Isomeren und dessen Additionssalzen mit einer pharmazeutisch annehmbaren Säure oder Base.

5. Verfahren nach einem der Ansprüche 1 bis 3 zur Herstellung von (2S , 3R)AHPA - (S)Lys - (S)PHI - (S)Arg - OH, dessen Isomeren und dessen Additionssalzen mit einer pharmazeutisch annehmbaren Säure oder Base.

6. Verfahren nach einem der Ansprüche 1 bis 3 zur Herstellung von AHPA - (S)Lys - (S)ABO - (S)Arg - OH, dessen Isomeren und dessen Additionssalzen mit einer pharmazeutisch annehmbaren Säure oder Base.

7. Verfahren nach einem der Ansprüche 1 bis 3 zur Herstellung von N-[2-Hydroxy-3(R)-amino-butyryl]-(S)Lys - (S)ABO - (S)Arg - OH, dessen Isomeren und dessen Additionssalzen mit einer pharmazeutisch annehmbaren Säure oder Base.

8. Verfahren nach einem der Ansprüche 1 bis 3 zur Herstellung von (3-Phenyl-2 (S) -amino-propyl) - (S)Lys - (S)ABO - (S)Arg - OH, dessen Isomeren und dessen Additionssalzen mit einer pharmazeutisch annehmbaren Säure oder Base.

9. Verfahren nach einem der Ansprüche 1 bis 3 zur Herstellung von (2 -Hydroxy-3(S)-amino-4-phenyl-butyl) - (S)Lys - (S)ABO - (S)Arg - OH, dessen Isomeren und dessen Additionssalzen mit einer pharmazeutisch annehmbaren Säure oder Base.

10. Verfahren nach einem der Ansprüche 1 bis 3 zur Herstellung von (2-Hydroxy-3(R)-amino-4-phenyl-butyl) - (S)Lys - (S)ABO - (S)Arg - OH, dessen Isomeren und dessen Additionssalzen mit einer pharmazeutisch annehmbaren Säure oder Base.

11. Verfahren zur Herstellung einer pharmazeutischen Zubereitung enthaltend als Wirkstoffmindestens eine Verbindung nach einem der Ansprüche 1 bis 10 in Kombination mit einem oder mehreren inerten, nichttoxischen, pharmazeutisch annehmbaren Bindemitteln oder Trägermaterialien.